# FASCICULE DE BREVET EUROPEEN

(11) **EP 0 906 326 B1**
(45) Date de publication et mention de la délivrance du brevet: **21.11.2001**
(21) Numéro de dépôt: 97928316.5
(22) Date de dépôt: 10.06.1997
(51) Int. Cl.: C07H 17/075, A61K 31/70, C07H 7/02, C07H 15/04, C07D 311/00

(54) **NOUVEAUX DERIVES AROMATIQUES SUBSTITUES PAR UN RIBOSE, LEUR PROCEDE DE PREPARATION ET LEUR APPLICATION COMME MEDICAMENTS**
AROMATISCHE RIBOSE-SUBSTITUIERTE DERIVATE, VERFAHREN ZU IHRER HERSTELLUNG UND IHRER VERWENDUNG ALS ARZNEIMITTEL
NOVEL AROMATIC DERIVATIVES SUBSTITUTED BY A RIBOSE, THEIR METHOD OF PREPARATION AND APPLICATION AS MEDICINE

(30) Priorité: 11.06.1996 FR 9607207
(43) Date de publication de la demande: 07.04.1999
(73) Titulaire: Aventis Pharma S.A., 92160 Antony (FR)
(72) Inventeur: KLICH, Michel, F-93250 Villemomble (FR); LAURIN, Patrick, F-93100 Montreuil (FR); MUSICKI, Branislav, F-75013 Paris (FR); SCHIO, Laurent, F-93130 Noisy le Sec (FR)
(86) Numéro de dépôt international: FR9701022
(87) Numéro de publication internationale: WO9747634

(56) Documents cités:
- US-A- 4 226 978

## Description

La présente invention concerne de nouveaux dérivés aromatiques, substitués par un ribose, leur procédé de préparation et leur application comme médicaments.

L'invention a pour objet les composés de formule (I) : 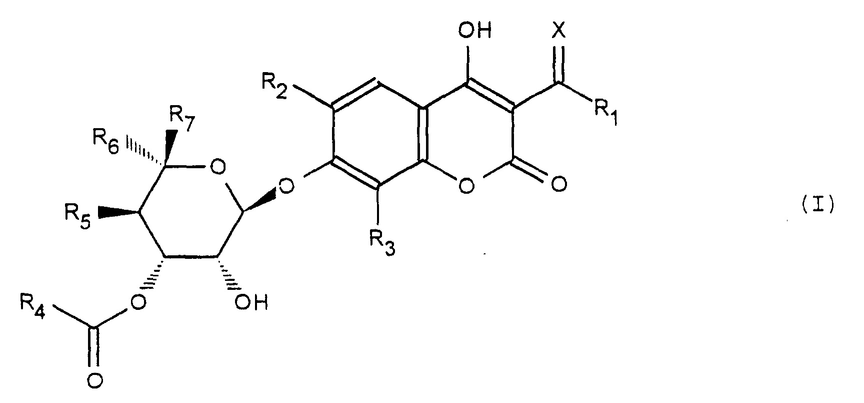 dans laquelle :
R₁ représente un atome d'hydrogène, un radical hydroxyle, un radical alkyle, alkényle ou alkynyle éventuellement interrompu par un atome d'oxygène, de soufre ou d'azote, renfermant jusqu'à 12 atomes de carbone linéaire, ramifié ou cyclique, éventuellement substitué par un ou plusieurs atomes d'halogène, un ou plusieurs radicaux OH, C≡N, NO₂, 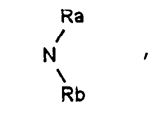 dans lequel Ra et Rb, identiques ou différents, représentent un atome d'hydrogène, un radical alkyle renfermant jusqu'à 8 atomes de carbone, ou Ra et Rb forment avec l'atome d'azote auquel ils sont liés un hétérocycle renfermant éventuellement un autre hétéroatome choisi parmi l'azote, le soufre ou l'oxygène, ou R₁ représente un radical alkoxy renfermant jusqu'à 8 atomes de carbone, éventuellement substitué par un ou plusieurs des substituants indiqués ci-dessus,
ou R₁ représente un radical NRcRd dans lequel Rc et Rd, identiques ou différents représentent un atome d'hydrogène ou un radical alkyle éventuellement interrompu par un atome d'oxygène, de soufre ou d'azote, renfermant jusqu'à 12 atomes de carbone, éventuellement substitué par un ou plusieurs des substituants indiqués ci-dessus, ou Rc et Rd forment ensemble avec l'atome d'azote auquel ils sont liés un hétérocycle renfermant éventuellement un autre hétéroatome choisi parmi l'azote, le soufre ou l'oxygène,
X représente un atome d'oxygène, ou un radical N-Nalc₁ ou NOalc₂ dans lequel alc₁ et alc₂ représentent un radical alkyle renfermant jusqu'à 12 atomes de carbone éventuellement substitué par un ou plusieurs atomes d'halogène, par un ou plusieurs radicaux 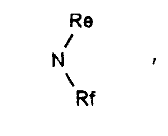 dans lequel Re et Rf, identiques ou différents l'un de l'autre représentent un atome d'hydrogène, un radical alkyle renfermant jusqu'à 8 atomes de carbone, éventuellement substitué, ou Re et Rf pouvant former ensemble avec l'atome d'azote auquel ils sont liés un hétérocycle pouvant renfermer en outre un atome d'oxygène, de soufre et un autre atome d'azote,
R₂ représente un atome d'hydrogène ou un atome d'halogène,
R₃ représente un atome d'hydrogène, un radical alkyle, renfermant jusqu'à 8 atomes de carbone ou un atome d'halogène, R₄ représente un radical 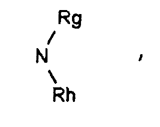 dans lequel Rg et Rh identiques ou différents l'un de l'autre, représentent un atome d'hydrogène, un radical alkyle linéaire, ramifié, cyclique, renfermant jusqu'à 8 atomes de carbone, un radical aryle ou hétéroaryle, éventuellement substitué, ou Rg et Rh forment avec l'atome d'azote auquel ils sont liés un hétérocycle pouvant renfermer en outre un atome d'oxygène, de soufre et un autre atome d'azote, ou R₄ représente un radical aryle ou hétéroaryle éventuellement substitué par un ou plusieurs atomes d'halogène, un ou plusieurs radicaux hydroxyle, un ou plusieurs radicaux alkyle ou alkoxy renfermant jusqu'à 8 atomes de carbone,
R₅ représente un atome d'hydrogène, un radical O-alkyle renfermant jusqu'à 4 atomes de carbone,
R₆ un radical alkyle ou CH₂-O-alkyle, dans lequel alkyle représente un radical alkyle renfermant jusqu'à 8 atomes de carbone,
R₇ représente un atome d'hydrogène ou un radical alkyle renfermant jusqu'à 8 atomes de carbone, ainsi que leurs sels.

Comme exemples de sels on peut citer les sels de sodium, de potassium, de lithium, de calcium ou de magnésium, les sels obtenus avec des bases azotées comme triméthylamine, la triéthylamine, la méthylamine, la propylamine, la N,N-diméthyléthanolamine et la tris (hydroxyméthyl) méthylamine.

Comme exemples de sels on peut également citer les sels formés avec les acides acétique, propionique, trifluoroacétique, maléique, tartrique, méthanesulfonique, benzènesulfonique, p-toluènesulfonique, chlorhydrique, bromhydrique, iodhydrique, sulfurique, phosphorique et spécialement les acides stéarique, éthylsuccinique ou laurylsulfonique.

Dans la définition des substituants :
- le radical alkyle, alkényle ou alkynyle est de préférence un radical méthyle, éthyle, propyle, isopropyle, n-butyle, isobutyle, terbutyle, décyle ou dodécyle, vinyle, allyle, éthynyle, propynyle, cyclobutyle, cyclopentyle ou cyclohexyle,
- l'halogène est de préférence le fluor ou le chlore, ou le brome,
- le radical aryle est de préférence le radical phényle,
- le radical hétérocyclique est de préférence le radical pyrrolyle pyrrolidinyle, pyridyle, pyrazinyle, pyrimidyle, pipéridinyle, pipérazinyle, quinuclidinyle, oxazolyle, isoxazolyle, morpholinyle, indolyle, imidazolyle, benzimidazolyle, triazolyle, thiazolyle, azétidinyle, aziridinyle.

Parmi les composés préférés de l'invention, on peut citer les composés dans lesquels R₂ représente un atome d'hydrogène, ceux dans lesquels R₃ représente un radical méthyle, ceux dans lesquels R₆ représente un radical méthyle, ceux dans lesquels R₇ représente un atome d'hydrogène ou un radical méthyle et ceux dans lesquels R₅ représente un radical OCH₃.

L'invention a tout spécialement pour objet les composés de formule (I), dans lesquels R₄ représente un radical 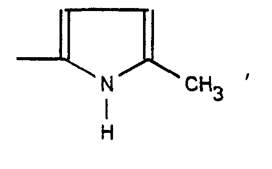 ou encore ceux dans lesquels R₄ représente un radical NH-cyclopropyle.

Parmi les composés de l'invention, on peut citer tout particulièrement les composés de formule (I) dans lesquels X représente un atome d'oxygène, ceux dans lesquels X représente un radical NOR, R représentant un radical alkyle, éventuellement substitué par un ou plusieurs atomes d'halogène et éventuellement interrompu par un atome d'oxygène, d'azote, de soufre et portant éventuellement un radical hétérocyclique éventuellement substitué, par exemple ceux dans lesquels X représente un radical NOCH₃, on peut également citer comme composés préférés les composés de formule (I) dans lesquels R₁ représente un radical alkyle éventuellement interrompu par un atome d'oxygène ou de soufre, un radical O-alkyle, éventuellement interrompu par un atome d'oxygène ou de soufre, un radical NH₂, par exemple les composés dans lesquels R₁ est un radical CH₃, 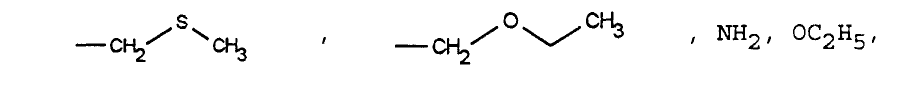 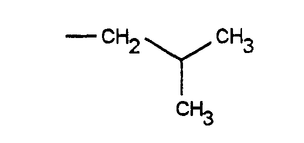 et de préférence les composés de formule (I) dans lesquels R₁ représente un radical méthyle ou O-éthyle.

Parmi les composés préférés de l'invention, on peut citer tout spécialement les composés dont la préparation est donnée ci-après dans la partie expérimentale et notamment les composés suivants :
- l'acide 5-méthyl-1H-pyrrole-2-carboxylique 3'-ester de 3-acétyl-7-((6-déoxy-5-C-méthyl-4-O-méthyl-alpha-L-lyxo-hexopyranosyl) oxy)-4-hydroxy-8-méthyl-2H-1-benzopyran-2-one,
- l'acide 5-méthyl-1H-pyrrole-2-carboxylique 3'-ester de 7-((6-déoxy-5-C-méthyl-4-O-méthyl-alpha-L-lyxo-hexopyranosyl) oxy)-4-hydroxy-8-méthyl-2-oxo-2H-1-benzopyran-3-carboxylate d'éthyle,
- l'acide 5-méthyl-1H-pyrrole-2-carboxylique 3'-ester de 7-((6-déoxy-5-C-méthyl-4-O-méthyl-alpha-L-lyxo-hexopyranosyl) oxy)-4-hydroxy-3-(1-(méthoxyimino) éthyl)-8-méthyl-2H-1-benzopyran-2-one,
- l'acide 5-méthyl-1H-pyrrole-2-carboxylique 3'-ester de 7-((6-déoxy-5-C-méthyl-4-O-méthyl-alpha-L-lyxo-hexopyranosyl) oxy)-3-(éthoxyacétyl)-4-hydroxy-8-méthyl-2H-1-benzopyran-2-one,
- l'acide 5-méthyl-1H-pyrrole-2-carboxylique 3'-ester de 3-(cyclopropylcarbonyl)-7-((6-deoxy-5-C-méthyl-4-O-méthylalpha-L-lyxo-hexopyranosyl) oxy)-4-hydroxy-8-méthyl-2H-1-benzopyran-2-one,
- l'acide 5-méthyl-1H-pyrrole-2-carboxylique 3'-ester de 7-((6-déoxy-5-C-méthyl-4-O-méthyl-alpha-L-lyxo-hexopyranosyl) oxy)-4-hydroxy-8-méthyl-2-oxo-2H-1-benzopyran-3-carboxamide.

Les produits de formule générale (I) possèdent une très bonne activité antibiotique sur les bactéries gram^{⊕} telles que les staphylocoques, les streptocoques, les pneumocoques, les entérocoques, listeria, anaerobies.

Les composés de l'invention peuvent donc être utilisés comme médicaments dans le traitement des infections à germes sensibles et, notamment, dans celui des staphylococcies, telles que les septicémies à staphylocoques, staphylococcies malignes de la face ou cutanées, pyodermites, plaies septiques ou suppurantes, furoncles, anthrax, phlegmons, érysipèles et acné, staphylococcies telles que les angines aigües primitives ou post-grippales, broncho-pneumonie, suppuration pulmonaires, les streptococcies telles que les angines aigües, les otites, les sinusites, la scarlatine, les pneumococcies telles que les pneumonies, les bronchites et la diphtérie. Les produits de la présente invention sont également actifs contre les infections dues à des germes comme Haemophilus influenzae.

L'invention a donc pour objet les composés de formule (I) à titre de médicaments.

L'invention a plus spécialement pour objet à titre de médicaments les composés indiqués ci-dessus comme composés préférés.

L'invention a également pour objet les compositions pharmaceutiques renfermant comme principe actif au moins un des médicaments définis ci-dessus.

Ces compositions peuvent être administrées par voie buccale, rectale, parentérale ou par voie locale en application topique sur la peau et les muqueuses, mais la voie d'administration préférée est la voie buccale.

Elles peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 3000 mg par jour par voie orale ou injectable, chez l'adulte pour les produits préférés.

L'invention a également pour objet un procédé caractérisé en ce que l'on soumet un composé de formule (II) : 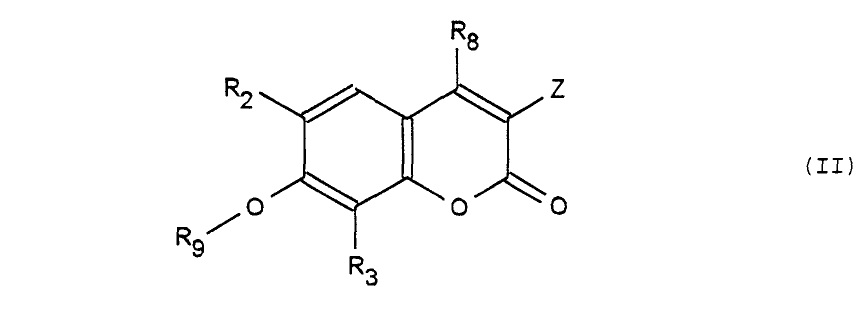 dans laquelle R₈ représente un radical hydroxyle libre ou bloqué, Z représente un atome d'hydrogène ou un radical 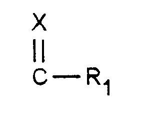 X, R₁, R₂ et R₃ conservent leur signification précédente, OR₉ représente un radical hydroxyle libre ou bloqué,
à l'action d'un composé de formule (III) : 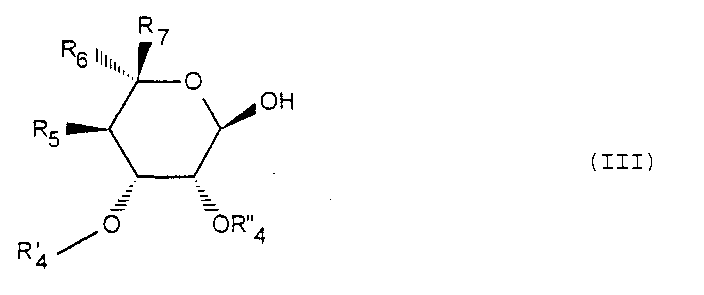 dans lequel R₅, R₆ et R₇ conservent leur signification précédente, OR'₄ représente un radical hydroxyle bloqué, R"₄ représente un atome d'hydrogène ou bien R'₄ et R"₄ forment ensemble avec les atomes de carbone auxquels ils sont joints un cycle 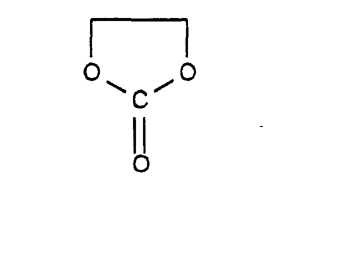 pour obtenir le composé de formule (IV) : 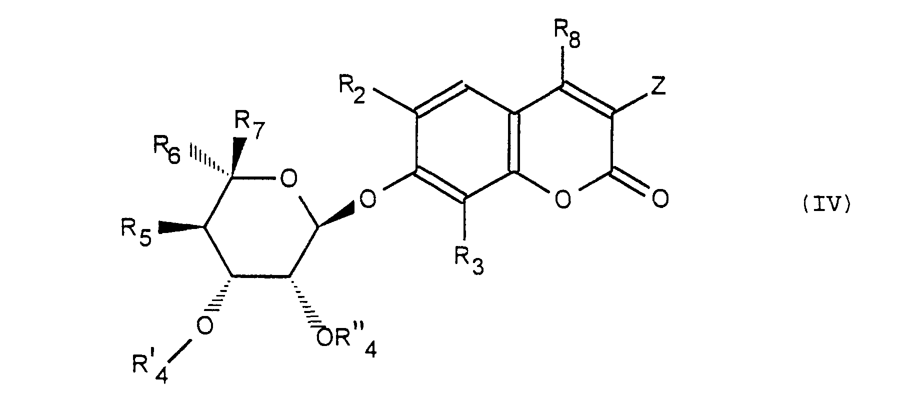 dans lequel les substituants conservent leur signification précédente,
puis soumet le composé de formule (IV) ainsi obtenu aux étapes suivantes, en totalité ou en partie :
- libération de l'hydroxyle en 4 après blocage éventuel de l'hydroxyle du sucre en α de OR'₄,
- si Z représente un atome d'hydrogène introduction du radical 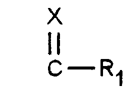 après blocage éventuel des hydroxyles pouvant réagir,
- introduction du radical 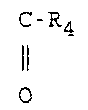 par substitution de ce radical au radical R'₄,
- modification du radical X.

Les produits de départ des composés de l'invention, à savoir les produits de formules (II) et (III) sont des produits nouveaux, des exemples de préparation de ces composés sont donnés ci-après dans la partie expérimentale.

Les composés de formule (IV) obtenus lors de la mise en oeuvre du procédé de l'invention sont nouveaux.

L'invention a donc pour objet à titre de produits chimiques nouveaux les composés de formules (II), (III) et (IV).

Dans un mode de réalisation préféré du procédé de l'invention :
- la réaction entre les composés de formules (II) et (III) a lieu en présence de d'azodicarboxylate de dialkyle comme l'azodicarboxylate de diéthyle ou de diisopropyle,
- la libération éventuelle de l'hydroxyle en 4 de la coumarine, est réalisée par hydrogénolyse ou par isomérisation puis hydrolyse,
- les autres hydroxyles protégés sont libérés par hydrolyse acide par exemple, en présence d'acide paratoluène sulfonique,
- l'introduction du radical 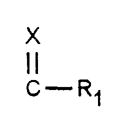 quand Z représente de l'hydrogène, se fait par acylation puis transposition,
- la glycosilation a lieu par réaction de Mitsunobu.
- les autres modifications sont réalisées dans des conditions classiques.

Les exemples suivants illustrent l'invention sans toutefois la limiter.

### PREPARATION 1 : 3-(5-méthyl-1H-pyrrole-2-carboxylate) de 6-déoxy-5-C-méthyl-4-O-méthyl-L-lyxohexopyranose

### STADE A : 6-déoxy-5-C-méthyl-4-O-méthyl-L-lyxo-hexopyranoside de phénylméthyle

On fait barboter à 20~22°C, pendant 2 heures, un courant d'acide chlorhydrique gaz dans une suspension renfermant 80 g de 6-déoxy 5-C-méthyl-4-O-méthyl-L-lyxohexopyranose et 400 ml d'alcool benzylique. On ajoute 120 ml d'eau déminéralisée et ajoute 40 g de carbonate de sodium, puis 240 ml d'acétate d'éthyle. On décante et extrait à l'acétate d'éthyle. On réunit les phases organiques et lave avec une solution saturée de chlorure de sodium. On sèche, essore, rince et distille sous agitation et sous vide de 45-50 mb. On distille l'alcool benzylique sous vide de 2 mb. On obtient 118,8 g d'un produit que l'on purifie par chromatographie sur silice en éluant avec le mélange chlorure de méthylène méthanol (95-5). On obtient ainsi 109,9 g de-produit recherché.

### STADE B : 2,3-O-carbonyl-6-déoxy-5-C-méthyl-4-O-méthyl-L-lyxo-hexopyranoside de phénylméthyle

On ajoute à 20~22°C, 67,5 g de 1,1-carbonyldiimidazole dans une solution renfermant 109 g de produit préparé au stade A et 1,1 l de dichloro-1-2-éthane. On porte au reflux pendant 2 heures. On ramène la température à 20~22°C puis amène à sec sous pression réduite à 25~30°C. On obtient 200,8 g de produit que l'on purifie par chromatographie sur silice en éluant avec le mélange chlorure de méthylène-méthanol (99-1). On obtient 91,6 g de produit recherché.

| Spectre I.R. : | |
|---|---|
| Cétone | 1813 cm⁻¹ |
| benzyle | 1498 cm⁻¹ |

Le 2-méthyl-1H-pyrrole utilisé au stade C a été préparé comme suit :

On ajoute 750 g de potasse caustique pure dans une suspension renfermant 5 l d'éthylène glycol et 370 g de 2-carboxaldéhyde pyrrole. On ajoute ensuite en 30 minutes 544 cm³ d'hydrate d'hydrazine à 64 %. On porte au reflux pendant 1 h 30 et ajoute 2 l d'eau déminéralisée puis verse dans un mélange eau-glace. On extrait au chlorure de méthylène, sèche, essore, rince et amène à sec. On obtient 270,3 g de produit que l'on purifie par distillation sous pression de 15 millibars. On récupère 227 g de produit recherché.
Teb = 46~47°C sous 15 millibars.

### STADE C : 3-(5-méthyl-1H-pyrrole-2-carboxylate) de 6-déoxy-5-C-méthyl-4-O-méthyl-L-lyxo-hexopyranoside de phénylméthyle

On ajoute en une heure à 0~2°C, une solution renfermant 60,2 g de 2-méthyl-1H-pyrrole dans 460 ml d'éther éthylique dans 248 ml d'une solution 3M de bromure de méthylmagnésium dans l'éther. On maintient le mélange réactionnel sous agitation à 0~2°C pendant 30 minutes et ajoute en 15 minutes 460 cm³ de toluène déthiophéné. On agite pendant 15 minutes à 0 ± 2°C et introduit en 45 minutes une solution de 91,3 g de produit préparé au stade B et 460 ml de toluène déthiophéné. On maintient l'agitation pendant 2 heures à 0 ± 2°C. On verse dans une solution aqueuse de chlorure d'ammonium. On décante, extrait à l'acétate d'éthyle, lave, sèche, rince et amène à sec sous pression réduite. On obtient 134,6 g de produit que l'on purifie par chromatographie sur silice en éluant avec le mélange chlorure de méthylène-acétone (8-2), puis (9-1). On obtient ainsi le produit recherché.

### STADE D : 3-(5-méthyl-1H-pyrrole-2-carboxylate) de 6-déoxy-5-C-méthyl-4-O-méthyl-L-lyxchexopyranose

On ajoute 14,7 g de palladium à 10 % sur charbon actif dans une solution renfermant 72,4 g de produit préparé au stade C et 1,45 l d'éthanol 100 dénaturé. On maintient sous pression d'hydrogène pendant 1 heure à 60~62°C. On laisse revenir à la température ambiante. On ajoute à nouveau 1,5 g de palladium à 10 % sur charbon actif. On agite sous pression d'hydrogène pendant 1 heure. On ramène à 20~22°C. On essore, filtre, rince, amène à sec et obtient 57 g de produit recherché.

### PREPARATION 2 : 7-hydroxy-8-méthyl-4-(2-propényloxy)-2H-1-benzopyran-2-one

### STADE A : 1-[2-hydroxy-3-méthyl-4-[(tétrahydro-2H-pyran-2-yl) oxy] phényl éthanone

On refroidit à 8°C un mélange renfermant 200 g de 1-[2,4-dihydroxy-3-méthyl] phényl éthanone et 1,2 l d'éther éthylique. On ajoute 200 ml de dihydro-2H-pyranne et 1 g d'APTS. On laisse revenir à la température ambiante. On maintient l'agitation pendant 3 heures et ajoute 253 mg d'APTS. On verse le produit obtenu sur 400 ml d'une solution aqueuse molaire de phosphate acide de potassium. On décante, lave à l'eau et sèche. On évapore et obtient 302,5 g de produit recherché brut que l'on purifie ainsi : on dilue le produit dans 2 l de chlorure de méthylène, lave la phase organique à l'ammoniaque diluée au dixième, puis à la saumure, sèche, filtre et évapore à sec. On obtient 272,55 g de produit recherché

### STADE B : 4-hydroxy-8-méthyl-7-[(tétrahydro-2H-pyran-2-yl) oxy]-2H-1-benzopyran-2-one

On chauffe à 90°C, un mélange renfermant 750 ml de toluène, 129,9 g de produit préparé au stade A et 126 ml de carbonate de diéthyle dans 620 ml de toluène. On ajoute 52 g d'hydrure de sodium à 55 % dans l'huile en maintenant la température à 90°C. On maintient sous agitation à 90°C et laisse revenir à la température ambiante. On ajoute 10 ml d'alcool éthylique. On essore et rince à l'éther éthylique, puis essore. On verse sur 1 l de solution aqueuse molaire de phosphate acide de sodium. On essore et rince à l'eau, à l'acétone et à l'éther. On obtient un produit que l'on sèche à 50°C en présence de P₂O₅. On obtient 141,89 g de produit recherché.

### STADE C : 8-méthyl-4-(2-propényloxy)-7-[(tétrahydro-2H-pyran-2-yl) oxy]-2H-1-benzopyran-2-one

On ajoute à 0°C, 9,45 ml d'azocarboxylate de diéthyle dans un mélange renfermant 13,814 g de produit préparé au stade précédent, 4,07 ml d'alcool allylique, 15,74 g de triphénylphosphine et 150 ml de dichlorométhane. On maintient sous agitation pendant 15 minutes à 0°C puis 2 heures à la température ambiante. On ajoute à nouveau 5,25 g de triphénylphosphine, 1,36 ml d'alcool allylique et 3,15 ml d'azodicarboxylate de diéthyle. On agite 2 heures à la température ambiante. On concentre et chromatographie le produit obtenu sur silice en éluant avec le mélange hexane-acétate d'éthyle (3-1). On obtient 7,85 g du produit.

### STADE D : 7-hydroxy-8-méthyl-4-(2-propényloxy)-2H-1-benzopyran-2-one

On agite pendant 6 heures à la température ambiante un mélange renfermant 7,80 g de produit préparé au stade précédent dans 150 ml de tétrahydrofuranne auquel on ajoute 100 ml d'une solution 1M d'HCl. On ajoute ensuite une solution saturée de chlorure de sodium et extrait à l'acétate d'éthyle. On sèche la phase organique sur sulfate de magnésium et concentre à sec. On sèche et obtient 4,40 g de produit recherché. rf = 0,26 hexane acétate d'éthyle (1-1).

### EXEMPLE 1 : Acide 5-méthyl-1H-pyrrole-2-carboxylique-3'-ester de 7-((6-déoxy-5-C-méthyl-4-O-méthyl-alpha-L-lyxo-hexopyranosyl) oxy) -4-hydroxy-8-méthyl-3-((méthylthio) acétyl)-2H-1-benzopyran-2-one

### STADE A : Acide 5-méthyl-1H-pyrrole-2-carboxylique-3'-ester de 7-[(6-déoxy-5-C-méthyl-4-O-méthyl-alpha-L-lyxo-hexopyranosyl) oxy]-8-méthyl-4-(2-propényloxy)-2H-1-benzopyran-2-one

On mélange 5 g de produit de la préparation 1, 4,65 g de produit de la préparation 2 et 5,26 g de triphénylphosphine et 500 ml de dichlorométhane. On ajoute ensuite à 0°C, 4 ml de diisopropylazodicarboxylate. On agite 1 heure à la température ambiante. On ajoute 2,19 g de triphénylphosphine et 1,65 ml d'azodicarboxylate de diéthyle. On agite 1 heure à la température ambiante et ajoute à nouveau 2,19 g de triphénylphosphine et 1,65 ml de diisopropylazodicarboxylate. On lave avec une solution aqueuse de dihydrogénophophate de sodium, et avec de la saumure. On sèche, filtre et concentre à sec. On chromatographie sur silice en éluant avec le mélange hexane-acétate d'éthyle (60-40). On obtient d'une part 3,51 g de produit A que l'on triture dans l'éther éthylique. On essore, sèche et obtient 3,0 g de produit recherché brut.

On obtient d'autre part 3,24 g de produit B, que l'on chromatographie sur silice en éluant avec le mélange chlorure de méthylène, acétate d'éthyle, 90-10 puis 80-20, puis avec le mélange chlorure de méthylène, acétate d'éthyle, tétrahydrofuranne (70-20-10), et obtient 0,88 g de produit que l'on triture sous ultrasons dans l'éther éthylique. On essore et obtient le produit recherché brut.

On réunit les deux quantités de produit recherché brut, les triture dans l'éther éthylique, essore et sèche. On obtient 3,625 g de produit recherché.

### STADE B : Acide 5-méthyl-1H-pyrrole-2-carboxylique-3'-ester de 7-[(6-déoxy-5-C-méthyl-4-O-méthyl-2-O-(tétrahydro-2H-pyran-2-yl)-alpha-L-lyxo-hexopyranosyl) oxy]-S-méthyl-4-(2-propényloxy)-2H-1-benzopyran-2-one

On ajoute 3,77 ml de dihydro-(2H)-pyranne, 200 mg d'acide paratoluènesulfonique (APTS) et 11 g de produit obtenu au stade précédent. On agite à la température ambiante pendant 2 heures. On traite le milieu réactionnel avec une solution saturée d'hydrogénocarbonate de sodium. On extrait au chlorure de méthylène. On réunit et sèche les phases organiques. On évapore le solvant à sec, triture le résidu dans un mélange hexane-acétate d'éthyle (3-1). On essore, sèche et obtient 8,40 g de produit recherché.

### STADE C : Acide 5-méthyl-1H-pyrrole-2-carboxylique-3'-ester de 7-[(6-déoxy-5-C-méthyl-4-O-méthyl-2-O-(tétrahydro-2H-pyran-2-yl)-alpha-L-lyxo-hexopyranosyl) oxy]-4-hydroxy-8-méthyl-2H-1-benzopyran-2-one

On ajoute à 0°C, 5,88 ml d'isopropylamine et 1,67 g de tétrakistriphénylphosphinepalladium dans une solution de 8,90 g de produit préparé au stade précédent et 90 ml de tétrahydrofuranne. On agite le mélange réactionnel à 0°C pendant 20 minutes et le verse sur un mélange de 50 ml d'une solution aqueuse d'hydrogénosulfate de sodium et de 100 ml d'un mélange hexane-acétate d'éthyle 1-2. On extrait la phase aqueuse avec un mélange hexane-acétate d'éthyle (1-2). On réunit les phases organiques et les sèche. On évapore les solvants et purifie le résidu par chromatographie sur silice en éluant avec le mélange chlorure de méthylène-méthanol (95-5). On obtient 2,50 g de produit recherché. rf = 0,22 CH₂Cl₂-CH₃OH (95-5).

### STADE D : Acide 5-méthyl-1H-pyrrole-2-carboxylique-3'-ester de 7-[(6-déoxy-5-C-méthyl-4-O-méthyl-2-O-(tétrahydro-2H-pyran-2-yl)-alpha-L-lyxo-hexopyranosyl) oxy]-4-hydroxy-8-méthyl-3-[(méthylthio) acétyl]-2H-1-benzopyran-2-one

On prépare une solution renfermant 400 mg du produit préparé au stade précédent et 5 ml de dichlorométhane anhydre. On ajoute 67 *µ*l d'acide 2-méthylthioacétique, 280 mg de 4-diméthylaminopyridine et 147 mg de chlorhydrate de N-(3-diméthylaminopropyl)-N'-éthylcarbodiimide. On maintient l'agitation pendant 18 heures à la température ambiante. On dilue avec 100 ml d'acétate d'éthyle. On lave avec une solution aqueuse d'hydrogénosulfate de sodium, avec de l'eau et avec de la saumure. On sèche, filtre et concentre à sec. On obtient 401 mg de produit que l'on purifie par chromatographie en éluant avec le mélange chloroforme méthanol (98-2). On sèche et obtient 324 mg de produit recherché. rf = 0,68 éluant = chlorure de méthylène-méthanol (95-5).

### STADE E : Acide 5-méthyl-1H-pyrrole-2-carboxylique-3'-ester de 7-[(6-déoxy-5-C-méthyl-4-O-méthyl-alpha-L-lyxo-hexopyranosyl) oxy]-4-hydroxy-8-méthyl-3-((méthylthio) acétyl)-2H-1-benzopyran-2-one

On ajoute 60 mg d'acide paratoluènesulfonique (APTS) dans une solution renfermant 293 mg de produit préparé au stade précédent et 10 ml de méthanol. On agite pendant 5 heures à la température ambiante. On dilue le milieu réactionnel avec un mélange acétate d'éthyle-hexane (67-33) et lave avec une solution diluée de bicarbonate de sodium. On rince à l'eau puis à la saumure. On sèche et évapore à sec. On obtient 227 mg de produit que l'on chromatographie sur silice en éluant avec le mélange chloroforme-méthanol (93-7). On recueille 171 mg de produit recherché brut que l'on chromatographie sur silice en éluant avec le mélange chloroforme-méthanol (94-6). On isole ainsi le produit recherché.
rf = 0,42 CHCl₃-CH₃OH (94-6).
RMN du proton (300 MHz, DMSO-d₆, ppm) δ
1,06 (s, 3H), 1,31 (s, 3H), 2,11 (s, 3H), 2,23 (s, 3H), 2,25 (s, 3H), 3,47 (s, 3H), 3,66 (d, 1H, J = 10 Hz), 3,92 (AB, 2H, J = 14,0 Hz), 4,19 (s, 1H), 5,48 (dd, 1H, J = 3,0 et 10,0 Hz), 5,70 (d, 1H, J = 2,5 Hz), 5,74 (m, 1H), 5,93 (m, 1H), 6,79 (m, 1H), 7,26 (d, 1H, J = 9,0 Hz), 7,94 (d, 1H, J = 9,0 Hz), 11,66 (sl, 1H).

### EXEMPLE 2 : Acide 5-méthyl-1H-pyrrole-2-carboxylique-3'-ester de 7-((6-déoxy-5-C-méthyl-4-O-méthyl-alpha-L-lyxo-hexopyranosyl) oxy)-3-(éthoxyacétyl)-4-hydroxy-8-méthyl-2H-1-benzopyran-2-one

### STADE A : Acide 5-méthyl-1H-pyrrole-2-carboxylique-3'-ester de 7-[(6-déoxy-5-C-méthyl-4-O-méthyl-2-O-(tétrahydro-2H-pyran-2-yl)-alpha-L-lyxo-hexopyranosyl) oxy]-3-(éthoxyacétyl)-4-hydroxy-8-méthyl-2H-1-benzopyran-2-one

On ajoute 55 *µ*l d'acide 2-éthoxyacétique dans une solution renfermant 300 mg du produit préparé au stade C de l'exemple précédent, 210 mg de 4-diméthylaminopyridine (DMAP) et 110 mg de chlorhydrate de N-(3-diméthylaminopropyl)-N'-éthylcarbodiimide. On agite 18 heures à la température ambiante. On dilue le milieu réactionnel avec 100 ml d'acétate d'éthyle. On lave avec une solution aqueuse à 10 % d'hydrogénosulfate de sodium, avec de l'eau puis avec de la saumure. On sèche la phase organique, filtre et concentre. On obtient 362 mg de produit que l'on purifie par chromatographie sur silice en éluant avec le mélange chlorure de méthylène-méthanol 97,5-2,5. On évapore les solvants et sèche sous pression réduite le produit obtenu. On obtient ainsi 265 mg de produit recherché. rf = 0,30, éluant chlorure de méthylène-méthanol (95-5).

### STADE B : Acide 5-méthyl-1H-pyrrole-2-carboxylique-3'-ester de 7-((6-déoxy-5-C-méthyl-4-O-méthyl-alpha-L-lyxo-hexopyranosyl) oxy)-3-(éthoxyacétyl)-4-hydroxy-8-méthyl-2H-1-benzopyran-2-one

On ajoute 50 mg d'acide p-toluènesulfonique dans une solution renfermant 245 mg du produit obtenu au stade A et 10 ml de méthanol. On agite 5 heures à la température ambiante. On dilue avec un mélange acétate d'éthyle-hexane et lave avec une solution diluée de bicarbonate de sodium. On rince à l'eau, à la saumure, sèche et évapore à sec. On obtient 163 mg de produit que l'on chromatographie sur silice en éluant avec le mélange chlorure de méthylène-méthanol (92-8). On obtient 116 mg de produit. rf = 0,20 chloroforme-méthanol (94-6).
RMN du proton (300 MHz, DMSO-d₆, ppm) δ
1,07 (s, 3H), 1,17 (t, 3H, J = 7,0 Hz), 1,30 (s, 3H), 2,22 (s, 3H), 2,26 (s, 3H), 3,56 (q, 2H, J = 7,0 Hz), 3,66 (d, 1H, J = 10,0 Hz), 4,18 (m, 1H), 4,71 (sl, 2H), 5,48 (dd, 1H, J = 3,0 et 10,0 Hz), 5,66 (sl, 1H), 5,72 (d, 1H, J = 5,0 Hz), 5,93 (m, 1H), 6,78 (m, 1H), 7,21 (d, 1H) J = 9,0 Hz), 7,90 (d, 1H, J = 9,0 Hz), 11,65 (sl, 1H), 15,25 (très large, 1H).

### EXEMPLE 3 : (Z) acide 5-méthyl-1H-pyrrole-2-carboxylique-3'-ester de 7-((6-déoxy-5-C-méthyl-4-O-méthyl-alpha-L-lyxohexopyranosyl) oxy)-4-hydroxy-3-(1-hydroxy-2-(2-pyridinyl) éthényl)-8-méthyl-2H-1-benzopyran-2-one

### STADE A : (Z) acide 5-méthyl-1H-pyrrole-2-carboxylique-3'-ester de 7-[(6-déoxy-5-C-méthyl-4-O-méthyl-2-O-(tétrahydro-2H-pyran-2-yl)-alpha-L-lyxo-hexopyranosyl) oxy] -4-hydroxy-3-(1-hydroxy-2-(2-pyridinyl) éthényl)-8-méthyl-2H-1-benzopyran-2-one

En opérant comme au stade A de l'exemple précédent à partir de 300 mg du produit du stade C de l'exemple 1 et de 100 mg de chlorhydrate d'acide 2-pyridylacétique, on a obtenu 135 mg de produit recherché.
rf = 0,64 CH₂Cl₂/THF (50-50)
rf = 0,45 CH₂Cl₂/MeOH (94-6).

### STADE B : (Z) acide 5-méthyl-1H-pyrrole-2-carboxylique-3'-ester de 7-((6-déoxy-5-C-méthyl-4-O-méthyl-alpha-L-lyxohexopyranosyl) oxy)-4-hydroxy-3-(1-hydroxy-2-(2-pyridinyl) éthényl)-8-méthyl-2H-1-benzopyran-2-one

On opère comme au dernier stade de l'exemple 1, et obtient 29 mg de produit recherché, à partir de 135 mg de produit préparé au stade A.
rf = 0,34 éluant CH₂Cl₂-MeOH (94-6).
RMN du proton (300 MHz, DMSO-d₆, ppm) δ
1,09 (s, 3H), 1,30 (s, 3H), 2,23 (s, 3H), 2,25 (s, 3H), 3,48 (s, 3H), 3,66 (d, 1H, J = 10,0 Hz), 4,16 (m, 1H), 5,49 (dd, 1H, J = 3,0 et 10,0 Hz), 5,60 (d, 1H, J = 2,0 Hz), 5,93 (m, 1H), 6,78 (m, 1H), 6,82 (s, 1H), 7,08 (dd, 1H, J = 5,0 et 7,5 Hz), 7,12 (d, 1H, J = 9,0 Hz), 7,62 (dl, 1H, J = 8,0 Hz), 7,80 (d, 1H, J = 9,0 Hz), 7,94 (dd, 1H, J = 7,5 et 8 Hz), 8,16 (d, 1H, J = 5 Hz), 11,65 (sl, 1H), 13,04 (m, 1H), 13,81 (m, 1H).

### PREPARATION 3 : 7-hydroxy 8-méthyl 2-oxo-4-(phénylméthoxy)-2H-1-benzopyran-3-carboxylate d'éthyle

### STADE A : 4-hydroxy-8-méthyl-2-oxo-7-[(tétrahydro-2H-pyran-2-yl) oxy]-2H-1-benzopyran-3-carboxylate d'éthyle

On ajoute 106,7 g de DMAP dans un mélange de 1,2 l de chlorure de méthylène et 120,65 g de produit préparé au stade B de la préparation 2. On refroidit le mélange obtenu au bain de glace et introduit en 50 minutes, 57,1 ml de chloroformate d'éthyle en gardant la température inférieure à 5°C. On ajoute à nouveau 26,6 g de DMAP et 21 ml de chloroformate d'éthyle, puis à nouveau 21,3 g de DMAP et 8,4 ml de chloroformate d'éthyle. On verse le milieu réactionnel sur 1 l d'une solution molaire aqueuse de phosphate acide de sodium 1M (NaHPO₄) puis du phosphate acide de sodium en poudre pour obtenir un pH égal à 6. On extrait la phase aqueuse au chlorure de méthylène. On réunit les phases organiques et les lave avec une solution d'HCl 1N. On lave à l'eau, décante, sèche, filtre et amène à sec.
On obtient 144,63 g du produit recherché.

### STADE B : 8-méthyl-2-oxo-4-(phénylméthoxy)-7-[(tétrahydro-2H-pyran-2-yl) oxy]-2H-1-benzopyran-3-carboxylate d'éthyle

On introduit en 1 h 30 mn à 0°C, 76 ml d'azodicarboxylate de dialkyle (DEAD) dans une solution renfermant 1,2 l de chlorure de méthylène, 120,90 g du produit préparé au stade précédent, 54 ml d'alcool benzylique et 109,1 g de triphénylphosphine. On filtre et verse le filtrat sur 500 ml d'une solution de phosphate acide de sodium 1M. On extrait au chlorure de méthylène, lave, sèche, filtre et amène à sec. On obtient 355,5 g de produit que l'on reprend dans le chlorure de méthylène. On laisse le mélange réactionnel pendant une nuit au réfrigérateur. On filtre, évapore le filtrat et le sèche. On obtient 324,7 g de produit que l'on reprend dans l'éther isopropylique, agite, filtre, rince à l'éther et sèche. On évapore sous pression réduite et obtient 251 g de produit brut que l'on purifie par chromatographie sur silice, en éluant au chlorure de méthylène puis avec le mélange chlorure de méthylène-acétate d'éthyle (90-10). On obtient ainsi le produit recherché.

### STADE C : 7-hydroxy 8-méthyl 2-oxo-4-(phénylméthoxy)-2H-1-benzopyran-3-carboxylate d'éthyle

On introduit 1 l d'une solution 1N d'HCl, dans une solution renfermant 90 g de produit préparé au stade précédent et 2 l de THF. On agite pendant 4 heures à la température ambiante. On ajoute 2 l de chlorure de méthylène, lave avec une solution aqueuse de bicarbonate de sodium à 10 %, puis à l'eau salée. On sèche et évapore à sec. On empâte le produit à l'éther, essore, rince et sèche sous pression réduite. On obtient 59,8 g de produit recherché.
rf = 0,15 CH₂Cl₂-CH₃CO₂Et (95-5).

| Spectre RMN DMSO | |
|---|---|
| H en 6 | 6,89 ppm |
| H de OH | 10,71 (s) ppm |
| H du méthoxy | 5,29 (s) ppm |
| H du CH₂CH₃ | 4,33 (a) |
| H du CH₂CH₃ | 1,29 (t) |
| H du méthyle en 8 | 2,16 (s) ppm |

### EXEMPLE 4 : Acide 5-méthyl-1H-pyrrole-2-carboxylique-3'-ester de 7-((6-déoxy-5-C-méthyl-4-O-méthyl-alpha-L-lyxo-hexopyranosyl) oxy)-4-hydroxy-8-méthyl-2-oxo-2H-1-benzopyran-3- carboxylate d'éthyle

### STADE A : Acide 5-méthyl-1H-pyrrole-2-carboxylique-3'-ester de 7-[(6-déoxy-5-C-méthyl-4-O-méthyl-alpha-L-lyxo-hexopyranosyl) oxy]-8-méthyl-2-oxo-4-(phénylméthoxy)-2H-1-benzopyran-3-carboxylate d'éthyle

En opérant comme à l'exemple 1 stade A, à partir des produits des préparations 1 et 3, on a obtenu le produit recherché, rf = 0,55 éther éthylique-hexane 1-2.

### STADE B : Acide 5-méthyl-1H-pyrrole-2-carboxylique-3'-ester de 7-((6-déoxy-5-C-méthyl-4-O-méthyl-alpha-L-lyxo-hexopyranosyl) oxy)-4-hydroxy-8-méthyl-2-oxo-2H-1-benzopyran-3-carboxylate d'éthyle

On ajoute 35 mg de palladium sur charbon à 10 % dans une solution renfermant 330 mg du produit préparé au stade A et 10 ml d'éthanol absolu. On agite pendant 5 heures le mélange à la température ambiante sous une légère pression d'hydrogène. On filtre, évapore l'éthanol à sec, triture le résidu sous ultrasons en présence d'éther isopropylique. On essore et sèche, on obtient 189,4 mg de produit recherché.
rf = 0,43 CH₂Cl₂-MeOH (91-9).
RMN du proton (300 MHz, DMSO-d₆, ppm) δ
1,07 (s, 3H), 1,30 (s, 3H), 1,32 (t, 3H, J = 7,0 Hz), 2,22 (s, 3H), 2,25 (s, 3H), 3,48 (s, 3H), 3,66 (d, 1H, J = 10,0 Hz), 4,18 (sl, 1H), 4,37 (q, 2H, J = 7,0 Hz), 5,48 (dd, 1H, J = 3,0 et 10,0 Hz), 5,66 (d, 1H, J = 2,0 Hz), 5,73 (sl, 1H), 5,93 (t, 1H, J = 3 Hz), 6,78 (t, 1H, J = 3,0 Hz), 7,23 (d, 1H, J = 9,0 Hz), 7,86 (d, 1H, J = 9,0 Hz), 11,66 (sl, 1H).

### EXEMPLE 5 : Acide 5-méthyl-1H-pyrrole-2-carboxylique-3'-ester de 3-acétyl-7-((6-déoxy-5-C-méthyl-4-O-méthyl-alpha-L-lyxohexopyranosyl) oxy)-4-hydroxy-8-méthyl-2H-1-benzopyran-2-one

### STADE A : 7-((6-déoxy-5-C-méthyl-4-O-méthyl-alpha-L-lyxohexopyranosyl) oxy)-8-méthyl-2-oxo-4-(phénylméthoxy)-2H-1-benzopyran-3-carboxylate d'éthyle

On ajoute 136 *µ*l d'acide acétique dans une solution renfermant 1 g du produit préparé au stade C de l'exemple 1, 764 mg de 4-diméthylaminopyridine, 433 mg de chlorhydrate de N-(3-diméthyl aminopropyl)-N'-éthylcarbodiimide et 20 ml de dichlorométhane. On agite le mélange réactionnel pendant 16 heures à la température ambiante et dilue dans du dichlorométhane. On lave la solution résultante avec une solution aqueuse d'hydrogénosulfate de sodium à 10 %. On sèche, filtre et concentre à sec. On triture le mélange réactionnel dans un mélange hexane-acétate d'éthyle (2-1). On essore et sèche. On obtient 634 mg du produit recherché. rf = 0,63 CH₂Cl₃-MeOH (94-6).

### STADE B : Acide 5-méthyl-1H-pyrrole-2-carboxylique-3'-ester de 3-acétyl-7-((6-déoxy-5-C-méthyl-4-O-méthyl-alpha-L-lyxohexopyranosyl) oxy)-4-hydroxy-8-méthyl-2H-1-benzopyran-2-one

On ajoute 80 mg d'acide paratoluènesulfonique dans une solution renfermant 568,4 mg du produit préparé au stade A, 20 ml de méthanol et 15 ml de dichlorométhane. On agite à la température ambiante pendant 4 heures. On verse le mélange réactionnel sur un mélange de dichlorométhane et de solution aqueuse saturée de bicarbonate de sodium. On extrait la phase aqueuse au dichlorométhane. On sèche, filtre et concentre à sec, sous pression réduite. On obtient 254 mg de produit recherché. rf = 0,39 CH₂Cl₃-MeOH (94-6).
RMN du-proton (300 MHz, DMSO-d₆, ppm) δ
1,06 (s, 3H), 1,30 (s, 3H), 2,22 (s, 3H), 2,24 (s, 3H), 2,66 (s, 3H), 3,47 (s, 3H), 3,66 (d, 1H, J = 9,5 Hz), 4,18 (sl, 1H), 5,48 (dd, 1H, J = 3 et 9,5 Hz), 5,69 (d, 1H, J = 2,5 Hz), 5,75 (d, 1H, J = 5 Hz), 5,93 (t, 1H, J = 3,0 Hz), 6,79 (t, 1H, J = 3,0 Hz), 7,25 (d, 1H, J = 8,5 Hz), 7,92 (d, 1H, J = 8 Hz), 11,66 (sl, 1H), 15,44 (sl, 1H).

### PREPARATION 4 : 7-hydroxy 8-méthyl 2-oxo-4-(phénylméthoxy)-2H-1-benzopyran-3-carboxylate de 2-méthylpropyle

### STADE A : carbonate de [8-méthyl-2-oxo-7-[(tétrahydro-2H-pyran-2-yl) oxy] -2H-1-benzopyran-4-yl] 2-méthylpropyle

On ajoute 6,96 ml de triéthylamine puis 3,57 ml de chloroformate d'isobutyle dans une solution renfermant 6,907 g de produit de la préparation 2 stade B, et 40 ml de tétrahydrofuranne. On agite pendant 1 heure à la température ambiante, dilue au tétrahydrofuranne et lave avec une solution aqueuse d'hydrogénosulfate de sodium à 10 %. On sèche et concentre à sec. On triture sous ultrasons en présence d'un mélange hexane-acétate d'éthyle (2-1). On essore et sèche. On obtient 7,475 g de produit. rf = 0,74 CH₂Cl₂-MeOH (94-6).

### STADE B : 4-hydroxy-8-méthyl-2-oxo-7-[(tétrahydro-2H-pyran-2-yl) oxy]-2H-1-benzopyran-3-carboxylate de 2-méthylpropyle

On ajoute 2,37 g de DMAP dans une solution renfermant 7,30 g de produit préparé au stade A dans 50 ml de dichlorométhane. On agite 5 heures à température ambiante. On dilue le mélange réactionnel avec du chlorure de méthylène et le lave avec une solution aqueuse d'hydrogénosulfate de sodium à 10 %, on sèche et concentre sous pression réduite. On chromatographie le produit obtenu sur silice en éluant avec le mélange hexane-acétate d'éthyle (1-1). On évapore les solvants et sèche. On obtient 3,0 g de produit recherché.
rf = 0,38 hexane-acétate d'éthyle (1-1).

### STADE C : 8-méthyl-2-oxo-4-(phénylméthoxy)-7-[(tétrahydro-2H-pyran-2-yl) oxy]-2H-1-benzopyran-3-carboxylate de 2-méthylpropyle

On ajoute 1,422 ml d'azodicarboxylate de diéthyle à 0°C dans un mélange renfermant 2,80 g de produit préparé au stade précédent, 928 *µ*l d'alcool benzylique et 2,34 g de triphénylphosphine. On agite 2 heures à la température ambiante. On rajoute 976 mg de triphénylphosphine et 585 *µ*l d'azodicarboxylate de diéthyle et agite pendant 2 heures à la température ambiante. On concentre, chromatographie sur silice en éluant avec le mélange hexane-acétate d'éthyle (2-1). On évapore les solvants et sèche. On obtient 2,18 g de produit recherché. rf = 0,25 hexane-acétate d'éthyle (2-1).

### STADE D : 7-hydroxy 8-méthyl 2-oxo-4-(phénylméthoxy)-2H-1-benzopyran-3-carboxylate de 2-méthylpropyle

On ajoute 35 ml d'une solution aqueuse d'acide chlorhydrique 1M dans une solution renfermant 2,00 g de produit préparé au stade précédent et 70 ml de tétrahydrofuranne. On agite 7 heures à la température ambiante. On dilue avec un mélange hexane-acétate d'éthyle (1-1). On sépare les phases et extrait la phase aqueuse avec un mélange hexane-acétate d'éthyle (1-1). On réunit les phases organiques et les lave avec une solution de dihydrogène phosphate de sodium 1M, et les sèche. On concentre à sec. Le résidu est trituré sous ultra-sons dans un mélange hexane-acétate d'éthyle (4-1). On essore, sèche et recueille 1,27 g de produit.
rf = 0,64 chlorure de méthylène-méthanol (94-6).

### EXEMPLE 6 : Acide 5-méthyl-1H-pyrrole-2-carboxylique-3'-ester de 7-[(6-déoxy-5-C-méthyl-4-O-méthyl-alpha-L-lyxo-hexopyranosyl) oxy]-4-hydroxy-8-méthyl-2-oxo-2H-1-benzopyran-3-carboxylate de 2-méthylpropyle

### STADE A : Acide 5-méthyl-1H-pyrrole-2-carboxylique-3'-ester de 7-[(6-déoxy-5-C-méthyl-4-O-méthyl-alpha-L-lyxo-hexopyranosyl) oxy]-8-méthyl-2-oxo-4-(phénylméthoxy)-2H-1-benzopyran-3-carboxylate de 2-méthylpropyle

En opérant comme à l'exemple 1, stade A, à partir du produit de la préparation 4 et du produit de la préparation 1, on a obtenu le produit recherché.

### STADE B : Acide 5-méthyl-1H-pyrrole-2-carboxylique-3'-ester de 7-[(6-déoxy-5-C-méthyl-4-O-méthyl-alpha-L-lyxo-hexopyranosyl) oxy]-4-hydroxy-8-méthyl-2-oxo-2H-1-benzopyran-3-carboxylate de 2-méthylpropyle

On ajoute 50 mg de palladium sur charbon à 10 % dans une solution renfermant 430 mg de produit préparé au stade A, 4 ml d'éthanol et 3 ml de tétrahydrofuranne. On agite pendant 3 heures sous atmosphère d'hydrogène et filtre. On évapore le solvant à sec. On triture le résidu sous ultrasons en présence d'éther isopropylique et essore. On sèche le produit, et obtient ainsi 279 mg de produit recherché.
RMN du proton (300 MHz, DMSO-d₆, ppm) δ
0,94 (d, 6H, J = 6,5 Hz), 1,10 (s, 3H), 1,29 (s, 3H), 1,92 (m, 2H), 2,17 (s, 3H), 2,25 (s, 3H), 3,47 (s, 3H), 3,64 (d, 1H, J = 10,0 Hz), 3,89 (d, 2H, J = 6,5 Hz), 4,15 (m, 1H), 5,48 (dd, 1H, J = 3,0 et 10,0 Hz), 5,54 (sl, 1H), 5,64 (d, 1H, J = 5.0 Hz), 5,92 (m, 1H), 6,77 (m, 1H), 6,99 (d, 1H, J = 9,0 Hz), 7,76 (d, 1H, J = 9,0 Hz), 11,66 (sl, 1H).

### EXEMPLE 7 : 7-((3-O-(aminocarbonyl)-6-déoxy-5-C-méthyl-4-O-méthyl-alpha-L-lyxo-hexopyranosyl) oxy]-4-hydroxy-8-méthyl-2H-1-benzopyran-3-carboxylate de 2-méthylpropyle

### STADE A : 7-[(6-déoxy-5-C-méthyl-4-O-méthyl-alpha-L-lyxohexopyranosyl) oxy]-8-méthyl-2-oxo-4-(phénylméthoxy)-2H-1-benzopyran-3-carboxylate de 2-méthylpropyle

En opérant comme à l'exemple 1, stade A, à partir du 6-déoxy 5-C-méthyl-4-O-méthyl-L-lyxohexopyranose et du 7-hydroxy-8-méthyl-2-oxo-4-(phénylméthoxy)-2H-1-benzopyran-3-carboxylate de 2-méthylpropyle, on a obtenu le produit recherché. rf = 0,22 hexane-acétate d'éthyle (1-2).

### STADE B : 7-[(2,3-O-carbonyl-6-déoxy-5-C-méthyl-4-O-méthylalpha-L-lyxo-hexopyranosyl) oxy]-8-méthyl-2-oxo-4-(phénylméthoxy)-2H-1-benzopyran-3-carboxylate de 2-méthylpropyle

On porte au reflux une solution renfermant 700 mg de produit préparé au stade A, 408 mg de carbonyldiimidazole et 10 ml de THF. On maintient au reflux pendant 30 minutes et dilue avec 50 ml d'un mélange hexane-acétate d'éthyle (1-2). On lave avec une solution aqueuse de dihydrogénophosphate de sodium 1M. On sèche, filtre et concentre à sec. On chromatographie le produit obtenu en éluant avec le mélange hexane-acétate d'éthyle (1-1). On obtient 530 mg de produit recherché. rf = 0,63 (hexane-acétate d'éthyle 1-2).

### STADE C : 7-[(2,3-O-carbonyl-6-déoxy-5-C-méthyl-4-O-méthylalpha-L-lyxo-hexopyranosyl) oxy]-4-hydroxy-8-méthyl-2-oxo-2H-1-benzopyran-3-carboxylate de 2-méthylpropyle

On agite à la température ambiante pendant une heure sous atmosphère d'hydrogène un mélange renfermant 4 ml d'éthanol, 2 ml de THF et 300 mg de produit préparé au stade précédent. On filtre, rince au THF le catalyseur, évapore à sec le produit obtenu, le triture sous ultrasons en présence d'éther éthylique. On essore et sèche. On obtient 180 mg de produit. rf = 0,52 hexane-acétate déthyle (1-1).

### STADE D : 7-((3-O-(aminocarbonyl)-6-déoxy-5-C-méthyl-4-O-méthyl-alpha-L-lyxo-hexopyranosyl) oxy]-4-hydroxy-8-méthyl-2H-1-benzopyran-3-carboxylate de 2-méthylpropyle

On ajoute 15 ml d'ammoniac liquide dans une solution renfermant 506 mg du produit préparé au stade C et 3 ml de THF. On laisse l'ammoniac s'évaporer. On met en solution dans un mélange THF-acétate d'éthyle-hexane (5-3-2). On lave avec une solution aqueuse d'hydrogénosulfate de sodium à 10 %, sèche et concentre à sec. On triture le résidu sous ultrasons en présence d'éther isopropylique. On essore et sèche. On obtient 310 mg de produit recherché. rf = 0,25 CH₂Cl₃MeOH (91-9).
RMN du proton (400 MHz, DMSO-d₆, ppm) δ
0,98 (d, 6H, J = 6,5 Hz), 1,02 (s, 3H), 1,27 (s, 3H), 2,01 (m, 1H), 2,18 (s, 3H), 3,46 (s, 3H), 3,47 (d, 1H, J = 10,0 Hz), 3,47 (s, 1H), 4,08 (tl, 1H, J = 2,5 Hz), 4,11 (d, 2H, J = 6,5 Hz), 5,14 (dd, 1H, J = 5 et 10,3 Hz), 5,57 (d, 1H, J = 2,5 Hz), 5,63 (s, 1H), 6,61 (sl, 2H), 7,19 (d, 1H, J = 9 Hz), 7,84 (d, 1H, J = 9 Hz).

### PREPARATION 5 : (E) 3-[1-((2-bromoéthoxy) imino) éthyl]-7-hydroxy-8-méthyl-4-(2-propényloxy)-2H-1-benzopyran-2-one

### STADE A : (E) 3-[1-((2-bromoéthoxy) imino) éthyl]-4-hydroxy-8-méthyl-7-[(tétrahydro-2H-pyran-2-yl) oxy] -2H-1-benzopyran-2-one

On chauffe au reflux pendant 1 heure un mélange de 3,183 g du produit préparé au stade A de la préparation 7, 4,418 g de bromhydrate de bromoéthyle O-hydroxylamine et 2,94 g d'acétate de potassium dans 20 cm³ d'éthanol. On évapore l'éthanol et dissout le résidu dans 100 ml de dichlorométhane en présence de 50 ml d'une solution aqueuse de dihydrogénophosphate de sodium. On sèche, évapore le chlorure de méthylène, chromatographie le résidu sur silice en éluant avec le mélange hexane-acétate d'éthyle (2-1). On évapore les solvants et sèche. On obtient 2,00 g de produit. rf = 0,57 CH₂Cl₂-MeOH (94-6).

### STADE B : (E) 3-[1-((2-bromoéthoxy) imino) éthyl]-7-hydroxy-8-méthyl-4-(2-propényloxy)-2H-1-benzopyran-2-one

On ajoute à 0°C, 914 *µ*l d'azodicarboxylate de diéthyle dans une solution renfermant 1,96 g de produit préparé au stade précédent, 360 *µ*l d'alcool allylique, 1,401 g de triphénylphosphine et 30 ml de dichlorométhane. On agite 2 heures à la température ambiante, ajoute à 0°C 180 *µ*l d'alcool allylique, 700 mg de triphénylphosphine et 457 *µ*l d'azodicarboxylate de diéthyle. On agite encore pendant 2 heures à la température ambiante, concentre et chromatographie sur silice en éluant avec un mélange hexane-acétate d'éthyle (4-1). On obtient 2,0 g de produit que l'on verse dans 40 ml de tétrahydrofuranne. On ajoute 20 ml d'une solution aqueuse d'acide chlorhydrique 1M et agite 2 heures à la température ambiante. On dilue avec 30 ml d'un mélange hexane-acétate d'éthyle 1-2. On réunit les phases organiques, les lave avec une solution aqueuse de dihydrogénophosphate de sodium, sèche, filtre et concentre. On triture sous ultrasons en présence d'un mélange hexane-acétate d'éthyle (3-1). On filtre et sèche. On obtient 1,17 g de produit recherché. rf = 0,28 hexane-acétate d'éthyle (4-1).

### EXEMPLE 8 : (E) acide 5-méthyl-1H-pyrrole-2-carboxylique-3'-ester de 3-[1-[(2-bromoéthoxy) imino] éthyl]-7-[(6-déoxy-5-C-méthyl-4-O-méthyl-alpha-L-lyxo-hexopyranosyl) oxy]-4-hydroxy-8-méthyl-2H-1-benzopyran-2-one

### STADE A : (E) acide 5-méthyl-1H-pyrrole-2-carboxylique-3'-ester de 3-[1-((2-bromoéthoxy) imino) éthyl]-7-[(6-déoxy-5-C-méthyl-4-O-méthyl-alpha-L-lyxo-hexopyranosyl) oxy]-8-méthyl-4-(2-propényloxy)-2H-1-benzopyran-2-one

On ajoute goutte à goutte à 0°C, 580 *µ*l d'azodicarboxylate de diéthyle dans un mélange renfermant 1,12 g de produit préparé à la préparation 5, stade A, 1,015 g de produit de la préparation 1, 890 mg de triphénylphosphine et 15 ml de dichlorométhane. On agite pendant 2 heures à la température ambiante et ajoute 450 mg de triphénylphosphine et 295 *µ*l d'azodicarboxylate de diéthyle. On agite pendant 1 heure, et ajoute à nouveau 300 mg de triphénylphosphine et 196 *µ*l d'azodicarboxylate de diéthyle. On agite 1 heure et concentre, chromatographie deux fois sur silice en éluant avec un mélange hexane-acétate d'éthyle (2-1). On évapore les solvants et sèche. On obtient 862 mg de produit recherché.

### STADE B : (E) acide 5-méthyl-1H-pyrrole-2-carboxylique-3'-ester de 3-[1-[(2-bromoéthoxy) imino] éthyl]-7-[(6-déoxy-5-C-méthyl-4-O-méthyl-alpha-L-lyxo-hexopyranosyl) oxy]-4-hydroxy-8-méthyl-2H-1-benzopyran-2-one

Le produit a été obtenu à partir du produit préparé au stade précédent en opérant comme à l'exemple 1 stade C
rf = 0,28 éther éthylique-hexane 2-1.
RMN du proton (300 MHz, DMSO-d₆, ppm) δ
1,08 (s, 3H), 1,30 (s, 3H), 2,24 (s, 3H), 2,25 (s, 3H), 2,28 (s, 3H), 3,47 (s, 3H), 3,65 (d, 1H, J = 9,5 Hz), 3,74 (t, 2H, J = 5,5 Hz), 4,17 (m, 1H), 4,48 (t, 2H, J = 5,5 Hz), 5,48 (dd, 1H, J = 3 et 9,5 Hz), 5,64 (d, 1H, J = 2,5 Hz), 5,71 (m, 1H), 5,93 (m, 1H), 6,78 (m, 1H), 7,21 (d, 1H, J = 9,0 Hz), 7,81 (d, 1H, J = 9,0 Hz), 11,66 (sl, 1H), 13,14 (ml, 1H).

### EXEMPLE 9 : (E) acide 5-méthyl-1H-pyrrole-2-carboxylique-3'-ester de 7-[(6-déoxy-5-C-méthyl-4-O-méthyl-alpha-L-lyxohexopyranosyl) oxy]-3-(1-(2-(diméthylamino) éthoxy) imino) éthyl-4-hydroxy-8-méthyl-2H-1-benzopyran-2-one

On ajoute 10 mg d'iodure de tétrabutylammonium, puis une solution de 53 mg de 4-diméthylamine dans 1 ml de DMF sur une solution renfermant 150 mg du produit de l'exemple 8 dans 2 ml de DMF anhydre. On agite le mélange réactionnel pendant 4 heures à la température ambiante. On verse sur un mélange de 50 ml de THF-AcOEt-hexane (2-2-1) et 50 ml d'eau. On extrait la phase aqueuse, lave les phases organiques, sèche et concentre à sec. On triture sous ultrasons en présence d'éther isopropylique. On essore et sèche, on obtient 55 mg de produit recherché.
rf = 0,10 CH₂Cl₂-CH₃OH (91-9)
RMN du proton (300 MHz, DMSO-d₆, ppm) δ
1,09 (s, 3H), 1,29 (s, 3H), 2,06 (s, 3H), 2,25 (s, 3H), 2,19 (s, 3H), 2,86 (s, 6H), 3,36 (1, 2H), 3,47 (s, 3H), 3,64 (d, 1H, J = 9,5 Hz), 4,15 (m, 1H), 4,34 (1, 2H), 5,48 (dd, 1H, J = 2,5 et 9,5 Hz), 5,63 (1, 1H), 5,93 (1, 1H), 6,78 (1, 1H), 7,00 (ml, 1H), 7,75 (ml, 1H).

### EXEMPLES 10, 11, 12, 13 :

En opérant comme à l'exemple 9, on a obtenu les produits suivants :
- (E) acide 5-méthyl-1H-pyrrole-2-carboxylique-3'-ester de 7-[(6-déoxy-5-C-méthyl-4-O-méthyl-alpha-L-lyxo-hexopyranosyl) oxy]-4-hydroxy-8-méthyl-3-(1-((2-(4-morpholinyl) éthoxy) imino) éthyl)-2H-1-benzopyran-2-one
   rf = 0,33 CH₂Cl₂-CH₃OH (91-9)
   RMN du proton (300 MHz, DMSO-d₆, ppm) δ
   1,10 (s, 3H), 1,30 (s, 3H), 2,03 (s, 3H), 2,19 (s, 3H), 2,22 (s, 3H), 3,25-3,42 (1, 4H), 3,47 (s, 3H), 3,63 (d, 1H, J = 9,5 Hz), 4,02 (1, 6H), 4,14 (sl, 1H), 4,34 (1, 2H), 5,48 (dd, 1H, J = 3 et 9,5 Hz), 5,54 (d, 1H, J = 2 H2), 5,64 (d, 1H, J = 4,5 Hz), 5,93 (t, 1H, J = 3,0 Hz), 6,77 (t, 1H, J = 3,0 Hz), 7,03 (d, 1H, J = 8,5 Hz), 7,75 (d, 1H, J = 8,5 Hz), 11,66 (sl, 1H).
- (E) acide 5-méthyl-1H-pyrrole-2-carboxylique-3'-ester de 7-[(6-déoxy-5-C-méthyl-4-O-méthyl-alpha-L-lyxo-hexopyranosyl) oxy]-4-hydroxy-3-[1-[[2-(4-hydroxy-1-pipéridinyl) éthoxy] imino] éthyl]-8-méthyl-2H-1-benzopyran-2-one
   rf = 0,05 CH₂Cl₂-CH₃OH (94-6)
   RMN du proton (300 MHz, DMSO-d₆, ppm) δ
   1,10 (s, 3H), 1,30 (s, 3H), 1,70-2,25 (m, 4H), 2,02 (s, 3H), 2,19 (s, 3H), 2,24 (s, 3H), 3,34 (m, 6H), 3,47 (s, 3H), 3,63 (d, 1H, J = 9,5 Hz), 3,87 (sl, 1H), 4,13 (m, 1H), 4,34 (sl, 2H), 5,00 (s, 1H), 5,48 (dd, 1H, J = 3,0 et 9,5 Hz), 5,53 (d, 1H, J = 2 Hz), 5,63 (d, 1H, J = 5,0 Hz), 5,92 (t, 1H, J = 3,0 Hz), 6,78 (t, 1H, J = 3,0 Hz), 6,98 (d, 1H, J = 9,0 Hz), 7,71 (d, 1H, J = 9,0 Hz), 11,66 (sl, 1H).
- (E) acide 5-méthyl-1H-pyrrole-2-carboxylique-3'-ester de 7-[(6-déoxy-5-C-méthyl-4-O-méthyl-alpha-L-lyxo-hexopyranosyl) oxy]-4-hydroxy-3-[1-[[2-[(1H-imidazol-2-yl) thio] éthoxy] imino] éthyl]-8-méthyl-2H-1-benzopyran-2-one
   rf = 0,20 CH₂Cl₂-CH₃OH (94-6)
   RMN du proton (300 MHz, DMSO-d₆, ppm) δ
   1,09 (s, 3H), 1,30 (s, 3H) 2,23 (s, 3H), 2,25 (s, 3H), 3,38 (m, 2H), 3,48 (s, 3H), 3,66 (d, 1H, J = 9,5 Hz), 4,17 (dd, 1H, J = 2,0 et 3,0 Hz), 4,37 (t, 2H, J = 5,5 Hz), 5,48 (dd, 1H, J = 3,0 et 9,5 Hz), 5,62 (d, 1H, J = 2 Hz), 5,93 (t, 1H, J = 3,0 Hz), 6,78 (t, 1H, J = 3,0 Hz), 7,16 (d, 1H, J = 9,0 Hz), 7,21 (s, 2H), 7,82 (d, 1H, J = 9,0 Hz), 11,66 (s, 1H).
- (E) acide 5-méthyl-1H-pyrrole-2-carboxylique-3'-ester de 7-[(6-déoxy-5-C-méthyl-4-O-méthyl-alpha-L-lyxo-hexopyranosyl) oxy]-4-hydroxy-8-méthyl-3-[1-[[2-(1H-1,2,4-triazol-1-yl) éthoxy] imino] éthyl]-2H-1-benzopyran-2-one
   rf = 0,36 CH₂Cl₂CH₃OH (91-9).
   RMN du proton (300 MHz, DMSO-d₆, ppm) δ
   1,07 (s, 3H), 1,30 (s, 3H), 2,14 (s, 3H), 2,25 (s, 3H), 3,47 (s, 3H), 3,66 (d, 1H, J = 9,5 Hz), 4,17 (s, 1H), 4,51 (m, 4H), 5,48 (dd, 1H, J = 2,5 et 9,5 Hz), 5,64 (d, 1H, J = 2 Hz), 5,72 (1, 1H), 5,93 (sl, 1H), 6,78 (t, 1H, J = 3,0 Hz), 7,21 (d, 1H, J = 9,0 Hz), 7,81 (d, 1H, J = 9,0 Hz), 8,01 (s, 1H), 8,54 (s, 1H), 11,66 (l, 1H).

### PREPARATION 6 : (E) 3-[2-éthoxy-1-(méthoxyimino) éthyl]-7-hydroxy-8-méthyl-4-(2-propényloxy)-2H-1-benzopyran-2-one

### STADE A : (E) 3-(éthoxyacétyl)-4-hydroxy-8-méthyl-7-[(tétrahydro-2H-pyran-2-yl) oxy]-2H-1-benzopyran-2-one

On ajoute 2,21 ml d'acide éthoxy acétique dans une solution renfermant 5,00 g de 4-hydroxy-8-méthyl-7-[(tétrahydro-2H-pyran-2-yl) oxy]-2H-1-benzopyran-2-one, 7,30 g de 4-diméthylaminopyridine et 3,816 g de chlorhydrate de N-(3-diméthylaminopropyl)-N'-éthylcarbodiimide. On agite ce mélange pendant 16 heures à la température ambiante. On dilue avec du dichlorométhane, lave avec une solution aqueuse de dihydrogénophosphate de sodium puis avec de la saumure. On sèche sur sulfate de magnésium, filtre et évapore à sec. On triture le produit obtenu dans un mélange hexane-acétate d'éthyle (4-1). On essore et sèche le produit obtenu. On obtient ainsi 5,40 g de produit recherché.

### STADE B : (E) 3-[2-éthoxy-1-(méthoxyimino) éthyl]-4-hydroxy-8-méthyl-7-[(tétrahydro-2H-pyran-2-yl) oxy]-2H-1-benzopyran-2-one

On chauffe au reflux pendant 2 heures un mélange de 2 g du produit obtenu au stade précédent, 922 mg de chlorhydrate de méthoxylamine et 1,62 g d'acétate de potassium et 20 ml d'éthanol. On évapore à sec l'éthanol. On chromatographie le produit obtenu sur silice en éluant avec un mélange hexane-acétate d'éthyle (1-1), puis avec le mélange chlorure de méthylène-méthanol 94-6. On évapore les solvants et sèche. On obtient 1,014 g du produit.
rf = 0,22 CH₂Cl₂-CH₃OH (94-6)

### STADE C : (E) 3-[2-éthoxy-1-(méthoxyimino) éthyl]-8-méthyl-4-(2-propényloxy)-7-[(tétrahydro-2H-pyran-2-yl) oxy]-2H-1-benzopyran-2-one

On ajoute à 0°C, 534 *µ*l d'azodicarboxylate de diéthyle dans un mélange renfermant 1 g de produit préparé au stade précédent, 208 *µ*l d'alcool allylique, 804 mg de triphénylphosphine et 15 ml de dichlorométhane. On agite pendant 2 heures à la température ambiante, ajoute 402 mg de triphénylphosphine, 104 *µ*l d'alcool allylique et 267 *µ*l d'azodicarboxylate de diéthyle. On agite pendant 2 heures à la température ambiante, concentre sous pression réduite et chromatographie sur silice en éluant avec un mélange hexane-acétate d'éthyle (2-1). On évapore les solvants, sèche et obtient 672 mg de produit. rf = 0,60 hexane-acétate d'éthyle (1-2).

### STADE D : (E) 3-[2-éthoxy-1-(méthoxyimino) éthyl]-7-hydroxy-8-méthyl-4-(2-propényloxy)-2H-1-benzopyran-2-one

On ajoute 12 ml d'une solution aqueuse d'acide chlorhydrique dans une solution renfermant 650 mg de produit précédent dans 12 ml de tétrahydrofuranne. On agite 6 heures à la température ambiante, on dilue avec 50 ml d'un mélange hexane-acétate d'éthyle (1-2). On extrait avec le mélange hexane-acétate d'éthyle (1-2). On réunit les phases organiques, les lave avec une solution de dihydrogénophosphate de sodium, sèche, filtre et concentre à sec. On triture le résidu sous ultrasons en présence d'un mélange hexane-acétate d'éthyle (3-1). On essore, sèche et obtient 410 mg de produit recherché.
rf = 0,50 hexane-acétate d'éthyle (1-1).

### EXEMPLE 14 : (E) acide 5-méthyl-1H-pyrrole-2-carboxylique-3'-ester de 7-[(6-déoxy-5-C-méthyl-4-O-méthyl-alpha-L-lyxohexopyranosyl) oxy]-3-(2-éthoxy 1-(méthoxyimino) éthyl)-4-hydroxy-8-méthyl-2H-1-benzopyran-2-one

### STADE A : (E) acide 5-méthyl-1H-pyrrole-2-carboxylique-3'-ester de 7-[(6-déoxy-5-C-méthyl-4-O-méthyl-alpha-L-lyxohexopyranosyl) oxy]-3-[2-éthoxy 1-(méthoxyimino) éthyl]-8-méthyl-4-(2-propényloxy)-2H-1-benzopyran-2-one

En opérant comme à l'exemple 1 stade 1, à partir du produit de la préparation 1 et du produit de la préparation 6, on a obtenu le produit recherché. rf = 0,41 hexane-acétate d'éthyle (1-1).

### STADE B : (E) acide 5-méthyl-1H-pyrrole-2-carboxylique-3'-ester de 7-[(6-déoxy-5-C-méthyl-4-O-méthyl-alpha-L-lyxohexopyranosyl) oxy]-3-(2-éthoxy 1-(méthoxyimino) éthyl)-4-hydroxy-8-méthyl-2H-1-benzopyran-2-one

On ajoute 31 mg de tétrakistriphénylphosphine palladium dans une solution renfermant 170 mg de produit préparé au stade précédent, 188 *µ*l de diisopropylamine et 5 ml de tétrahydrofuranne. On agite à 0°C pendant 20 minutes et verse sur 25 ml d'un mélange de solution aqueuse d'hydrogénosulfate de sodium à 10 % et 50 ml d'une solution d'hexane-acétate d'éthyle (1-4). On lave la phase organique, sèche, filtre et concentre à sec. On chromatographie le résidu sur silice, élue avec du dichlorométhane à 5 % de méthanol. On évapore les solvants et triture le résidu sous ultrasons, en présence d'éther éthylique. On essore et sèche. On obtient 72 mg de produit recherché.
rf = 0,41 hexane-acétate d'éthyle (1-1).
RMN du proton (300 MHz, DMSO-d₆, ppm) δ
0,96 (t, J = 7,0 Hz), 1,03 (t, J = 7,0 Hz), 1,08 (s), 1,29 (s), 2,20-2,30 (m), 3,30 (m), 3,47 (s), 3,50 (m), 3,65 (d, J = 10 Hz), 3,76 (s), 3,87 (s), 4,16 (m), 4,23 (s), 4,42 (s), 5,48 (dd, J = 3 et 10 Hz), 5,60 (m), 5,68 (d, J = 5,0 Hz), 5,93 (m), 6,78 (m), 7,10 (d, J = 9,0 Hz), 7,16 (d, J= 9,0 Hz), 7,77 (d, J = 9,0 Hz), 11,65 (sl).

### PREPARATION 7 : (E) 7-hydroxy-3-[1-(méthoxyimino) éthyl]-8-méthyl-4-(2-propényloxy)-2H-1-benzopyran-2-one

### STADE A : 3-acétyl-4-hydroxy-8-méthyl-7-[(tétrahydro-2H-pyran-2-yl) oxy]-2H-1-benzopyran-2-one

On ajoute 2,39 ml d'acide acétique dans une solution renfermant 10,00 g du produit du stade B de la préparation 2, 14,59 g de 4-diméthylaminopyridine et 7,63 g de chlorhydrate de N-(3-diméthylaminopropyl)-N'-éthylcarbodiimide. On agite pendant 5 heures à la température ambiante. On ajoute à nouveau 4,86 g de 4-diméthylaminopyridine et 3,82 g de chlorhydrate de N-(3-diméthylaminopropyl)-N'-éthylcarbodiimide, et agite pendant 16 heures supplémentaires.

On dilue au dichlorométhane et lave avec une solution aqueuse d'hydrogénosulfate de sodium à 10 %. On sèche, filtre et concentre à sec. On triture le résidu sous ultrasons en présence du mélange hexane-acétate d'éthyle (2-1). On essore et sèche. On obtient 10,04 g de produit recherché.
rf = 0,79 CH₂Cl₂-CH₃OH (95-5).

### STADE B : (E) 4-hydroxy-3-[1-(méthoxyimino) éthyl]-8-méthyl-7-[(tétrahydro-2H-pyran-2-yl) oxy]-2H-1-benzopyran-2-one

On chauffe 30 minutes à 80°C, un mélange de 1,7 g de produit préparé au stade précédent, 892 mg de chlorhydrate de méthoxylamine, 1,151 g d'acétate de potassium et 20 ml d'éthanol. On évapore à sec, et reprend le résidu au dichlorométhane en présence d'eau. On lave avec une solution aqueuse de dihydrogénophosphate de sodium (1M), sèche, filtre et évapore à sec. On triture sous ultrasons en présence d'un mélange hexane-acétate d'éthyle (4-1). On sèche. On obtient 1,60 g de produit.
rf = 0,28 CH₂Cl₂-CH₃OH (99,5-0,5)

### STADE C : (E) 3-[1-(méthoxyimino) éthyl]-8-méthyl-4-(2-propényloxy)-7-[(tétrahydro-2H-pyran-2-yl) oxy]-2H-1-benzopyran-2-one

On ajoute à 0°C, 937 *µ*l d'azodicarboxylate de diéthyle dans un mélange renfermant 1,56 g de produit préparé au stade B, 366 *µ*l d'alcool allylique, 1,413 g de triphénylphosphine et 15 ml de dichlorométhane. On agite pendant 2 heures à la température ambiante. On ajoute 706 mg de triphényl phosphine, 183 *µ* l d'alcool allylique et 468 *µ*l d'azodicarboxylate de diéthyle. On agite 2 heures supplémentaires à la température ambiante. On concentre le mélange réactionnel et le chromatographie sur silice en éluant avec le mélange hexane-acétate d'éthyle (4-1). On évapore les solvants et sèche. On obtient 1,00 g de produit recherché.
rf = 0,38 hexane-acétate d'éthyle (4-1).

### STADE D : (E) 7-hydroxy-3-[1-(méthoxyimino) éthyl]-8-méthyl-4-(2-propényloxy)-2H-1-benzopyran-2-one

On ajoute 10 ml d'une solution aqueuse d'acide chlorhydrique 1M, dans une solution renfermant 1 g de produit préparé au stade C et 20 ml de tétrahydrofuranne. On agite pendant 7 heures à la température ambiante. On dilue avec 50 ml d'un mélange hexane-acétate d'éthyle (1-1). On sépare les phases et extrait la phase aqueuse avec le mélange hexane-acétate d'éthyle (1-1). On réunit les phases organiques et les lave avec une solution de dihydrogénophosphate de sodium 1M, on sèche, filtre et concentre à sec. On triture le résidu avec un mélange hexane-acétate d'éthyle (4-1). On essore, sèche et recueille 770 mg de produit.

### EXEMPLE 15 : (E) acide 5-méthyl-1H-pyrrole-2-carboxylique-3'-ester de 7-[(6-déoxy-5-C-méthyl-4-O-méthyl-alpha-L-lyxohexopyranosyl) oxy]-4-hydroxy-3-(1-(méthoxyimino) éthyl)-8-méthyl-2H-1-benzopyran-2-one

### STADE A : (E) acide 5-méthyl-1H-pyrrole-2-carboxylique-3'-ester de 7-[(6-déoxy-5-C-méthyl-4-O-méthyl-alpha-L-lyxohexopyranosyl) oxy]-3-[1-(méthoxyimino) éthyl]-8-méthyl-4-(2-propényloxy)-2H-1-benzopyran-2-one

En opérant comme à l'exemple 1, à partir de 455 mg de produit préparé à la préparation 7 et de 539 mg du produit de la préparation 1, on obtient le produit recherché.
rf = 0,33 éluant héxane-acétate d'éthyle (1-1).

### STADE B : (E) acide 5-méthyl-1H-pyrrole-2-carboxylique-3'-ester de 7-[(6-déoxy-5-C-méthyl-4-O-méthyl-alpha-L-lyxohexopyranosyl) oxy]-4-hydroxy-3-(1-(méthoxyimino) éthyl)-8-méthyl-2H-1-benzopyran-2-one

On ajoute à 0°C, 460 *µ*l de diisopropylamine puis 75 mg de tétrakistriphénylphosphinepalladium, dans 6 ml de tétrahydrofuranne et 380 mg du produit du stade précédent. On agite 20 minutes à 0°C et verse sur un mélange de solution d'hydrogénosulfate de sodium à 10 % et de tétrahydrofuranne-hexane-acétate d'éthyle (1-1-1). On sépare les phases, extrait la phase aqueuse avec un mélange tétrahydrofuranne-hexane-acétate d'éthyle (1-1-1). On réunit les phases organiques, les sèche, filtre et concentre à sec. On triture le résidu sous ultrasons en présence d'éther isopropylique. On essore et sèche sous pression réduite. On obtient 180 mg de produit recherché.
rf = 0,35 CH₂Cl₂-CH₃OH (94-6)
RMN du proton (300 MHz, DMSO-d₆, ppm) δ
1,08 (s, 3H), 1,30 (s, 3H), 2,23 (s, 3H), 2,25 (s, 3H), 2,27 (s, 3H), 3,47 (s, 3H), 3,65 (d, 1H, J = 10,0 Hz), 3,95 (s, 3H), 4,17 (m, 1H), 5,48 (dd, 1H, J = 3,0 et 10,0 Hz), 5,63 (d, 1H, J = 2,5 Hz), 5,70 (d, 1H, J = 5,0 Hz), 5,93 (m, 1H), 6,78 (m, 1H), 7,20 (d, 1H, J = 9,0 Hz), 7,80 (d, 1H, J = 9,0 Hz), 11,65 (sl, 1H), 13,69 (ml, 1H).

### PREPARATION 8 : (E) 7-hydroxy-3-[(méthoxyimino) méthyl]-8-méthyl-4-(2-propényloxy)-2H-1-benzopyran-2-one

### STADE A : 4-hydroxy-8-méthyl-2-oxo-7-[(tétrahydro-2H-pyran-2-yl) oxy]-2H-1-benzopyran-3-carboxaldéhyde

On ajoute à la température ambiante 700 *µ*l d'acide formique dans une solution renfermant 75 ml de dichlorométhane, 5,0 g de produit préparé au stade B de la préparation 2, 6,625 g de 4-diméthylaminopyridine et 3,825 g de chlorhydrate de N-(3-diméthylaminopropyl)-N'-éthylcarbodiimide. On agite pendant 16 heures, dilue au dichlorométhane, lave avec une solution aqueuse d'hydrogénosulfate de sodium à 10 % puis avec une solution 1M. On sèche, filtre, évapore à sec. On triture sous ultrasons en présence d'éther, essore, sèche et recueille 4,04 g de produit recherché.

### STADE B : (E) 4-hydroxy-3-[(méthoxyimino) méthyl]-8-méthyl-7-[(tétrahydro-2H-pyran-2-yl) oxy]-2H-1-benzopyran-2-one

On chauffe au reflux pendant 1 heure un mélange renfermant 2,0 g du produit préparé au stade A, 1,08 g de chlorhydrate de méthoxylamine, 1,93 g d'acétate de potassium et 20 ml d'éthanol. On évapore à sec, dissout le produit obtenu dans un mélange de dichlorométhane et d'eau (100 ml-100 ml). On lave avec une solution diluée de phosphate acide de sodium et sèche. On évapore, triture le résidu sous ultrasons en présence d'éther éthylique, essore et sèche. On obtient 1,90 g de produit.

### STADE C : (E) 3-[(méthoxyimino) méthyl]-8-méthyl-4-(2-propényloxy)-7-[(tétrahydro-2H-pyran-2-yl) oxy]-2H-1-benzopyran-2-one

On ajoute à 0°C, 1,02 ml d'azodicarboxylate de diéthyle, dans un mélange renfermant 1,80 g du produit préparé au stade précédent, 441 *µ*l d'alcool allylique, 1,70 g de triphénylphosphine et 15 ml de dichlorométhane. On agite 2 heures à la température ambiante. On ajoute à 0°C, 845 mg du triphénylphosphine, 220 *µ*l d'alcool allylique et 564 *µ*l de DEAD. On agite pendant 2 heures, concentre sous pression réduite et chromatographie sur silice en éluant avec le mélange hexane-acétate d'éthyle (3-1). On évapore les solvants, sèche et obtient 1,60 g de produit.

### STADE D : (E) 7-hydroxy-3-[(méthoxyimino) méthyl]-8-méthyl-4-(2-propényloxy)-2H-1-benzopyran-2-one

On ajoute 20 ml d'une solution aqueuse d'acide chlorhydrique 1M dans une solution renfermant 1,60 g de produit préparé au stade précédent et 20 ml de THF. On agite pendant 6 heures à la température ambiante. On dilue la solution réactionnelle avec 30 ml d'un mélange hexane-acétate d'éthyle (1-2). On sépare les phases et extrait la phase aqueuse avec le mélange hexane-acétate d'éthyle (1-2). On lave les phases organiques avec une solution de dihydrogénophosphate de sodium 1M, sèche, filtre et concentre à sec. On triture le résidu sous ultrasons en présence d'hexane-acétate d'éthyle (3-1). On essore et sèche. On obtient 920 mg de produit recherché. rf = 0,47 hexane-acétate d'éthyle (1-2).

### EXEMPLE 16 : (E) acide 5-méthyl-1H-pyrrole-2-carboxylique-3'-ester de 7-[(6-déoxy-5-C-méthyl-4-O-méthyl-alpha-L-lyxohexopyranosyl) oxy]-4-hydroxy-8-méthyl-3-((méthoxyimino) méthyl)-2H-1-benzopyran-2-one

### STADE A : (E) acide 5-méthyl-1H-pyrrole-2-carboxylique-3'-ester de 7-[(6-déoxy-5-C-méthyl-4-O-méthyl-alpha-L-lyxohexopyranosyl) oxy]-3-[(méthoxyimino) méthyl]-8-méthyl-4-(2-propényloxy)-2H-1-benzopyran-2-one

En opérant comme à l'exemple 1, à partir du produit de la préparation 8 et du produit de la préparation 1, on a obtenu le produit recherché.
rf = 0,34 hexane-acétate d'éthyle (1-1).

### STADE B : (E) acide 5-méthyl-1H-pyrrole-2-carboxylique-3'-ester de 7- [(6-déoxy-5-C-méthyl-4-O-méthyl-alpha-L-lyxohexopyranosyl) oxy]-4-hydroxy-8-méthyl-3-((méthoxyimino) méthyl)-2H-1-benzopyran-2-one

On ajoute à 0°C, 87 mg de tétrakistriphénylphosphine palladium dans une solution renfermant 430 mg de produit préparé au stade A, 533 *µ*l de diisopropylamine et 10 ml de tétrahydrofuranne anhydre. On agite à 0°C pendant 20 minutes, on verse le mélange réactionnel sur 50 ml d'une solution aqueuse d'hydrogénosulfate de sodium et 50 ml d'un mélange hexane-acétate d'éthyle (1-2). On lave et sèche. On filtre et concentre à sec. On chromatographie sur silice, en éluant avec un mélange chlorure de méthylène-méthanol 95-5. On évapore les solvants, triture le résidu sous ultrasons en présence d'éther éthylique. On essore et sèche. On obtient 176 mg de produit recherché.
rf = 0,34 hexane-acétate d'éthyle 1-1.
RMN du proton (300 MHz, DMSO-d₆, ppm) δ
1,08 (s, 3H), 1,30 (s, 3H), 2,22 (s, 3H), 2,25 (s, 3H), 3,47 (s, 3H), 3,65 (d, 1H, J = 10,0 Hz), 3,90 (s, 3H), 4,16 (m, 1H), 5,48 (dd, 1H, J = 3,0 et 10,0 Hz), 5,61 (d, 1H, J = 2,0 Hz), 5,70 (d, 1H, J = 5,0 Hz), 5,93 (m, 1H), 6,78 (m, 1H), 7,16 (d, 1H, J = 9,0 Hz), 7,78 (d, 1H, J = 9,0 Hz), 8,35 (s, 1H), 11,66 (sl).

### EXEMPLE 17 : Acide cyclopropyl-carbamique-3'-ester de 3-acétyl-7-[(6-déoxy-5-C-méthyl-4-O-méthyl-alpha-L-lyxo-hexopyranosyl) oxy]-4-hydroxy-8-méthyl-2H-1-benzopyran-2-one

### STADE A : 7-[(6-déoxy-5-C-méthyl-4-O-méthyl-alpha-L-lyxohexopyranosyl) oxy]-8-méthyl-4-(2-propényloxy)-2H-1-benzopyran-2-one

On ajoute goutte à goutte 8,10 ml d'azodicarboxylate de diéthyle dans un mélange renfermant 8,50 g du produit de la préparation 2, 8,44 g de 6-déoxy 5-C-méthyl-4-O-méthyl-L-lyxohexopyranose, 11,52 g de triphénylphosphine, 100 ml de dichlorométhane, et 20 ml de tétrahydrofuranne. On maintient sous agitation pendant 2 heures à la température ambiante, et ajoute 5,70 g de triphénylphosphine et 4,05 ml d'azodicarboxylate de diéthyle. On maintient l'agitation pendant une nuit à la température ambiante. On concentre, purifie le produit par chromatographie sur silice, en éluant avec le mélange chlorure de méthylène-acétone (90-10). On évapore, sèche et recueille 8,304 g de produit recherché.

### STADE B : 7-[(2,3-O-carbonyl-6-déoxy-5-C-méthyl-4-O-méthylalpha-L-lyxo-hexopyranosyl) oxy]-8-méthyl-4-(2-propényloxy)-2H-1-benzopyran-2-one

On chauffe au reflux pendant 30 minutes un mélange de 2 g de produit préparé au stade A, et 1,995 g de carbonyldiimidazole. On refroidit et dilue avec 100 ml du mélange tétrahydrofuranne-acétate d'éthyle (1-1). On lave à l'eau, sèche, filtre et concentre. Le résidu est dissous dans du dichlorométhane puis chromatographié sur silice en éluant avec le mélange chlorure de méthylène-acétone (9-1). On évapore les solvants et obtient 1,32 g de produit recherché. rf = 0,69 CH₂Cl₂-CH₃COCH₃ (90-10).

### STADE C : 7-[(2,3-O-carbonyl-6-déoxy-5-C-méthyl-4-O-méthylalpha-L-lyxo-hexopyranosyl) oxy]-4-hydroxy-8-méthyl-2H-1-benzopyran-2-one

On ajoute 1 ml d'isopropylamine dans un mélange renfermant 1 g de produit préparé au stade précédent et 10 ml de tétrahydrofuranne. On ajoute 267 mg de tétrakistriphénylphosphinepalladium. On maintient l'agitation pendant 30 minutes à 0°C, ajoute 10 ml d'éther sulfurique et filtre. On obtient un produit que l'on filtre. On essore et rince avec un mélange éther éthylique-tétrahydrofuranne (1-1). On sèche et obtient 880 mg de produit que l'on verse dans 75 ml d'un mélange tétrahydrofuranne-acétate d'éthyle-hexane (2-2-1). On extrait la phase aqueuse avec le mélange de solvants. On réunit les phases organiques, les sèche, filtre et concentre. On obtient 726 mg de produit recherché.

### STADE D : 3-acétyl 7-[(2,3-O-carbonyl-6-déoxy-5-C-méthyl-4-O-méthyl-alpha-L-lyxo-hexopyranosyl) oxy]-4-hydroxy-8-méthyl-2H-1-benzopyran-2-one

On ajoute 225 *µ*l d'acide acétique dans une solution renfermant 1,29 g de produit obtenu au stade C, 1,344 g du 4-diméthylaminopyridine et 719 mg de chlorhydrate de N-(3-diméthylaminopropyl)-N'-éthyl carbodiimide et 15 ml de dichlorométhane. On agite 6 heures à la température ambiante. On dilue avec 50 ml de dichlorométhane. On lave avec une solution aqueuse d'hydrogénosulfate de sodium à 10 % et sèche. On évapore les solvants et chromatographie sur silice en éluant avec un mélange chlorure de méthylène-méthanol (98-2). On évapore les solvants et sèche. On obtient 1,18 g de produit recherché. rf = 0,73 chlorure de méthylène-méthanol (94-6).

### STADE E : Acide cyclopropyl-carbamique-3'-ester de 3-acétyl-7-[(6-déoxy-5-C-méthyl-4-O-méthyl-alpha-L-lyxo-hexopyranosyl) oxy)-4-hydroxy-8-méthyl-2H-1-benzopyran-2-one

On ajoute 783 *µ*l de DBUt et 362 *µ*l de cyclopropylamine dans une solution renfermant 1,137 g du produit préparé au stade précédent, et 5 ml de diméthylformamide. On maintient sous agitation pendant 3 heures et ajoute 181 *µ* l de cyclopropylamine. On agite pendant 3 heures. On verse sur 80 ml d'un mélange d'hexane-acétate d'éthyle 1-2, en présence d'une solution aqueuse d'hydrogénosulfate de sodium. On extrait avec un mélange hexane-acétate d'éthyle (1-2). On réunit les phases organiques, les lave, sèche et filtre. On évapore et sèche le résidu. On obtient 1,040 g de produit recherché.
rf = 0,58 CH₂Cl₂-MeOH (90-10).
RMN du proton (300 MHz, DMSO-d₆, ppm) δ
~ 0,43 (m, 2H), 0,60 (m, 2H), 1,04 (s, 3H), 1,18 (s, 3H), 2,19 (s, 3H), 2,65 (s, 3H), 3,12 (m, 1H), 3,45 (s, 3H), ~ 3,49 (m, 1H), ~ 4,02 (sl, 1H), 5,18 (dd, 1H, J = 3,0 et 10,0 Hz), 5,60 (sl, 1H), 7,21 (d, 1H, J = 9,0 Hz), 7,50 (sl, 1H), 7,90 (d, 1H, J = 9,0 Hz), 13,75 (sl, 1H).

### EXEMPLE 18 : (E) Acide cyclopropyl-carbamique-3'-ester de 7-[(6-déoxy-5-C-méthyl-4-O-méthyl-alpha-L-lyxo-hexopyranosyl) oxy]-4-hydroxy-3-(1-(méthoxyimino) éthyl)-8-méthyl-2H-1-benzopyran-2-one

On chauffe au reflux pendant 1 heure, un mélange de 100 mg de produit préparé à l'exemple précédent, 34 mg de chlorhydrate de méthoxylamine, 50 mg d'acétate de potassium et 2 ml d'éthanol. On évapore l'éthanol à sec et on reprend dans 40 ml de dichlorométhane. On lave avec une solution aqueuse de dihydrogénophosphate de sodium 1M et sèche sur sulfate de magnésium. On évapore le solvant et chromatographie sur plaques préparatives. On extrait et évapore les solvants puis dissout le produit obtenu dans l'éther éthylique et ajoute du n-pentane. On essore et sèche le produit obtenu. On obtient 56 mg de produit recherché. rf = 0,41 CH₂Cl₂-MeOH (94-6).
RMN du proton (300 MHz, DMSO-d₆, ppm) δ
0,44 (m, 2H), 0,60 (m, 2H), 1,04 (s, 3H), 1,26 (s, 3H), 2,20, (s, 3H), 2,27 (s, 3H), 3,45 (m, 1H), 3,45 (s, 3H), 3,95 (s, 3H), 4,09 (m, 1H), 5,19 (dd, 1H, J = 3,0 et 10,0 Hz), 5,55 (sl, 1H), 5,64 (d, 1H, J = 5,0 Hz), 7,16 (d, 1H, J = 9,0 Hz), 7,51 (m, 1H), 7,79 (d, 1H, J = 9,0 Hz), 13,67 (ml, 1H).

### EXEMPLE 19 : (E) acide cyclopropyl-carbamique-3'-ester de 7-[(6-déoxy-5-C-méthyl-4-O-méthyl-alpha-L-lyxo-hexopyranosyl) oxy] -3-(1-diméthylhydrazono) éthyl)-4-hydroxy-8-méthyl-2H-1-benzopyran-2-one

En opérant comme à l'exemple précédent en utilisant Me₂N-NH₂ au lieu de NH₂OMe.HCl, on a obtenu le produit recherché.
RMN du proton (300 MHz, DMSO-d₆, ppm) δ
0,43 (m, 2H), 0,60 (m, 2H), 1,04 (s, 3H), 1,25 (s, 3H), 2,16 (s, 3H), 2,68 (s, 6H), 2,76 (s, 3H), 3,44 (s, 3H), 3,48 (d, 1H, J = 10,0 Hz), 4,07 (sl, 1H), 5,18 (dd, 1H, J = 3,0 et 10,0 Hz), 5,51 (sl, 1H), 5,61 (d, 1H, J = 5,0 Hz), 7,05 (d, 1H, J = 9,0 Hz), 7,50 (sl, 1H), 7,78 (d, 1H, J = 9,0 Hz), 14,50 (sl, 1H).

### EXEMPLE 20 : 7-[(6-déoxy-5-C-méthyl-4-O-méthyl-3-O-(((5-méthyl-3-isoxazolyl) amino) carbonyl)-alpha-L-lyxo-hexopyranosyl) oxy]-4-hydroxy-8-méthyl-2-oxo-2H-1-benzopyran-3-carboxylate d'éthyle

### STADE A : 7-[(2,3-O-carbonyl-6-déoxy-5-C-méthyl-4-O-méthylalpha-L-lyxo-hexopyranosyl) oxy]-8-méthyl-2-oxo-4-(phénylméthoxy)-2H-1-benzopyran-3-carboxylate d'éthyle

On ajoute à 0°C, 5,33 ml de diisopropylazodicarboxylate dans un mélange renfermant 8,00 g de produit de la préparation 3, 5,207 g de 6-déoxy 5-C-méthyl-4-O-méthyl-L-lyxohexopyranose et 50 ml de dichlorométhane. On agite pendant 2 heures à la température ambiante. On ajoute 3,6 g de triphényl phosphine et 2,7 ml d'azodicarboxylate et agite pendant 2 heures. On concentre le milieu réactionnel et chromatographie sur silice en éluant avec le mélange hexane-acétate d'éthyle (2-3). On obtient 8,43 g de produit que l'on verse dans 70 ml de tétrahydrofuranne. On ajoute 5,17 g de carbonyldiimidazole et chauffe au reflux pendant 30 minutes. On refroidit la solution et la dilue avec 100 ml d'un mélange hexane-acétate d'éthyle (1-2). On lave avec une solution aqueuse de dihydrogénophosphate de sodium. On sèche, filtre et évapore les solvants. On chromatographie le produit obtenu sur silice en éluant avec le mélange hexane-acétate d'éthyle (1-1). On évapore les solvants et sèche. On obtient 6,48 g de produit recherché.

### STADE B : 7-[(2,3-O-carbonyl-6-déoxy-5-C-méthyl-4-O-méthylalpha-L-lyxo-hexopyranosyl) oxy]-4-hydroxy-8-méthyl-2-oxo-2H-1-benzopyran-3-carboxylate d'éthyle

On ajoute 600 mg de palladium sur charbon à 10 % dans une solution renfermant 6,00 g de produit préparé au stade A, 30 ml d'éthanol et 15 ml de tétrahydrofuranne. On agite ce mélange sous pression d'hydrogène pendant 6 heures, on filtre et rince au THF. On évapore les solvants et sèche. On obtient 4,572 g de produit recherché. rf = 0,59 CH₂Cl₂-MeOH (91-9).

### STADE C : 7-[(6-déoxy-5-C-méthyl-4-O-méthyl-3-O-(((5-méthyl-3-isoxazolyl) amino) carbonyl)-alpha-L-lyxo-hexopyranosyl) oxy]-4-hydroxy-8-méthyl-2-oxo-2H-1-benzopyran-3-carboxylate d'éthyle

On refroidit à -76°C une solution renfermant 254 mg de 5-méthylaminoisoxazole et 2 ml de tétrahydrofuranne anhydre. On ajoute à -76°C, 1,08 ml de n-butyllithium 1,6 M dans l'hexane. On maintient le mélange réactionnel sous agitation pendant 15 minutes. On ajoute une solution de 400 mg de produit préparé au stade précédent dans 2 ml de THF. On maintient le mélange réactionnel sous agitation à -76°C pendant 2 heures puis à -20°C pendant 40 minutes. On dilue avec 150 ml d'acétate d'éthyle à 20 % d'hexane, lave avec 70 ml d'une solution aqueuse de sulfate acide de sodium à 10 %, à l'eau et à la saumure, on sèche et évapore à sec après filtration. On recueille 504 mg de produit que l'on solubilise dans le minimum de dichlorométhane contenant 10 % de méthanol. On chromatographie sur silice en éluant avec le mélange chlorure de méthylène-éthanol 10 %. Après évaporation des solvants, on obtient 264 mg de produit que l'on chromatographie sur silice en éluant avec le mélange chlorure de méthylène-méthanol (94-6). On obtient 145 mg de produit. On obtient le produit recherché. rf = 0,21 CH₂Cl₂-CH₃OH (95-5). RMN du proton (300 MHz, DMSO-d₆, ppm) δ
1,05 (s, 3H), 1,28 (m, 3H), 1,28 (m, 3H), 2,19 (s, 3H), 2,37 (s, 3H), 3,49 (s, 3H), 3,57 (d, 1H, J = 10,0 Hz), 4,19 (sl, 1H), 4,32 (q, 2H, J = 7,0 Hz), 5,25 (dd, 1H, J = 3,0 et 10,0 Hz), 5,61 (sl, 1H), 5,83 (s, 1H), 6,56 (s, 1H), 7,16 (d, 1H, J = 9,0 Hz), 7,83 (d, 1H, 9,0 Hz).

En opérant comme à l'exemple précédent à partir du produit préparé au stade B de l'exemple précédent et des amines correspondants, on a obtenu les produits suivants :

### EXEMPLE 21 : 7-[(6-déoxy-5-C-méthyl-4-O-méthyl-3-O-(((2-thiazolyl) amino) carbonyl)-alpha-L-lyxo-hexopyranosyl) oxy]-4-hydroxy-8-méthyl-2-oxo-2H-1-benzopyran-3-carboxylate d'éthyle

rf = 0,07 CH₂Cl₂-CH₃OH (95-5)
RMN du proton (300 MHz, DMSO-d₆, ppm) δ
1,07 (s, 3H), 1,25 (t, 3H, J = 7,0 Hz), 1,29 (s, 3H), 2,18 (s, 3H), 3,51 (s, 3H), 3,57 (d, 1H, J = 10,0 Hz), 4,21 (m, 3H), 5,28 (dd, 1H, J = 3,0 et 10,0 Hz), 5,57 (sl, 1H), 5,80 (d, 1H, J = 5,0 Hz), 7,05 (dl, 1H, J = 9,0 Hz), 7,77 (d, 1H, J = 9,0 Hz), 7,22 (d, 1H, J = 3,5 Hz), 7,42 (d, 1H, J = 3,5 Hz), 11,95 (sl, 1H).

### EXEMPLE 22 : 7-[(6-déoxy-5-C-méthyl-4-O-méthyl-3-O-(((phénylméthyl) amino) carbonyl)-alpha-L-lyxo-hexopyranosyl) oxy]-4-hydroxy-8-méthyl-2-oxo-2H-1-benzopyran-3-carboxylate d'éthyle

rf = 0,30 CH₂Cl₂-CH₃OH (95-5)
RMN du proton (300 MHz, DMSO-d₆, ppm) δ
1,02 (s, 3H), 1,27 (s, 3H), 1,32 (t, 3H, J = 7,0 Hz), 2,17 (s, 3H), 3,48 (s, 3H), 3,51 (d, 1H, J = 10,0 Hz), 4,11 (sl, 1H), 4,21 (m, 2H), 4,37 (q, 2H, J = 7,0 Hz), 5,22 (dd, 1H, J = 3,0 et 10,0 Hz), 5,59 (d, 1H, J = 2,0 Hz), 5,71 (1, 1H), 7,21 (d, 1H, J = 9,0 Hz), 7,30 (ml, 5H), 7,85 (d, 1H, J = 9,0 Hz), 7,92 (t, 1H, J = 6,0 Hz).

### EXEMPLE 23 : 7-[(3-O-(((cyclopentyl)amino) carbonyl)-6-déoxy-5-C-méthyl-4-O-méthyl-alpha-L-lyxo-hexopyranosyl) oxy]4-hydroxy-8-méthyl-2-oxo-2H-1-benzopyran-3-carboxylate d'éthyle

rf = 0,32 CH₂Cl₂-CH₃OH (95-5)
RMN du proton (300 MHz, DMSO-d₆, ppm) δ
1,26 (s, 3H), 1,32 (s, 3H), 1,32 (t, 3H, J = 7,0 Hz), 1,40-1,90 (m, 8H), 2,18 (s, 3H), 3,46 (s, 3H), 3,50 (d, 1H, J = 10,0 Hz), 3,82 (m, 1H), 4,08 (1, 1H), 4,32 (q, 2H, J = 7,0 Hz), 5,19 (dd, 1H, J = 3,0 et 10,0 Hz), 5,58 (sl, 1H), 5,64 (1, 1H), 7,18 (d, 1H, J = 9,0 Hz), 7,33 (d, 1H, J = 7,0 Hz), 7,85 (d, 1H, J = 9,0 Hz).

### EXEMPLE 24 : 7-[(3-O-(aminocarbonyl)-6-déoxy-5-C-méthyl-4-O-méthyl-alpha-L-lyxo-hexopyranosyl) oxy]-4-hydroxy-8-méthyl-2-oxo-2H-1-benzopyran-3-carboxylate d'éthyle

rf = 0,06 CH₂Cl₂-CH₃OH (95-5)
RMN du proton (300 MHz, DMSO-d₆, ppm) δ
1,03 (s, 3H), 1,26 (s, 3H), 1,32 (t, 3H, J = 7,0 Hz), 2,18 (s, 3H), 3,47 (s, 3H), 3,48 (1, 1H), 4,08 (dd, 1H, J = 2,5 et 3,0 Hz), 4,37 (q, 2H, J = 7,0 Hz), 5,15 (dd, 1H, J = 3,0 et 10,0 Hz), 5,58 (d, 1H, J = 2,5 Hz), 6,55-6,75 (1, 2H), 7,19 (d, 1H, J = 9,0 Hz), 7,85 (d, 1H, J = 9,0 Hz).

### EXEMPLE 25 : 7-[(6-déoxy-5-C-méthyl-4-O-méthyl-3-O-(((2-pyridinylméthyl) amino) carbonyl)-alpha-L-lyxo-hexopyranosyl) oxy]-4-hydroxy-8-méthyl-2-oxo-2H-1-benzopyran-3-carboxylate d'éthyle

rf = 0,11 CH₂Cl₂-CH₃OH (95-5)
RMN du proton (300 MHz, DMSO-d₆, ppm) δ
1,02 (s, 3H), 1,28 (s, 3H), 1,32 (t, 3H, J = 7,0 Hz), 2,16 (s, 3H), 3,50 (s, 3H), 3,54 (d, 1H, J = 10,0 Hz), 4,11 (sl, 1H), 4,36 (m, 4H), 5,22 (dd, 1H , J = 3,0 et 10,0 Hz), 5,60 (d, 1H, J = 2,0 Hz), 5,73 (1, 1H), 7,19 (d, 1H, J = 9,0 Hz), 7,29 (t, 1H, J = 6,0 Hz), 7,36 (d, 1H, J = 8,0 Hz), 7,83 (m, 2H), 7,97 (t, 1H, J = 6,0 Hz), 8,22 (m, 1H).

### EXEMPLE 26 : 7-[(3-O-(cyclopropylamino) carbonyl)-6-déoxy-5-C-méthyl-4-O-méthyl-alpha-L-lyxo-hexopyranosyl) oxy]-4-hydroxy-8-méthyl-2-oxo-2H-1-benzopyran-3-carboxylate d'éthyle

rf = 0,32 CH₂Cl₂-CH₃OH (92-8)
RMN du proton (300 MHz, DMSO-d₆, ppm) δ
0,43 (m, 2H), 0,59 (m, 2H), 1,03 (s, 3H), 1,26 (s, 3H), 1,32 (t, 3H, J = 7,0 Hz), 2,19 (s, 3H), 3,45-3,48 (m, 2H), 3,44 (s, 3H), 4,09 (sl, 1H), 4,37 (q, 2H, J = 7,0 Hz), 5,17 (dl, 1H, J = 10,0 Hz), 5,58 (sl, 1H), 5,65 (m, 1H), 7,19 (d, 1H, J = 9,0 Hz), 7,50 (sl, 1H), 7,85 (d, 1H, J = 9,0 Hz).

### EXEMPLE 27 : 7-[(3-O-((cyclobutylamino) carbonyl)-6-déoxy-5-C-méthyl-4-O-méthyl-alpha-L-lyxo-hexopyranosyl) oxy]-4-hydroxy-8-méthyl-2-oxo-2H-1-benzopyran-3-carboxylate d'éthyle

rf = 0,38 CH₂Cl₂-CH₃OH (92-8)
RMN du proton (300 MHz, DMSO-d₆, ppm) δ
1,01 (s, 3H), 1,26 (s, 3H), 1,32 (t, 3H, J = 7,0 Hz), 1,58 (m, 2H), 1,93 (m, 2H), 2,15 (m, 2H), 2,17 (s, 3H), 3,46 (s, 3H), 3,28 (d, 1H, J = 10,0 Hz), 4,00 (m, 1H), 4,07 (sl, 1H), 4,36 (q, 2H, J = 7,0 Hz), 5,15 (dd, 1H, J = 3,0 et 10,0 Hz), 5,57 (d, 1H, J = 2,0 Hz), 5,67 (m, 1H), 7,18 (d, 1H, J = 9,0 Hz), 7,63 (d, 1H, J = 8,0 Hz), 7,85 (d, 1H, J = 9,0 Hz).

### EXEMPLE 28 : 7-[(6-déoxy-5-C-méthyl-4-O-méthyl-3-O-(((1-méthyléthyl) amino) carbonyl)-alpha-L-lyxo-hexopyranosyl) oxy]-4-hydroxy-8-méthyl-2-oxo-2H-1-benzopyran-3-carboxylate d'éthyle

rf = 0,30 CH₂Cl₂CH₃OH (92-8)
RMN du proton (300 MHz, DMSO-d₆, ppm) δ
1,03 (s, 3H), 1,09 (d, 6H, J = 6,5 Hz), 1,26 (s, 3H), 1,32 (t, 3H, J = 7,00 Hz), 2,18 (s, 3H), 3,46 (s, 3H), 3,50 (d, 1H, J = 10,0 Hz), 3,67 (m, 1H), 4,08 (sl, 1H), 4,37 (q, 2H, J = 7,0 Hz), 5,19 (dd, 1H, J = 3,0 et 10,0 Hz), 5,58 (d, 1H, J = 2,0 Hz), 5,65 (sl, 1H), 7,18 (d, 1H, J = 9,0 Hz), 7,22 (d, 1H, J = 7,5 Hz), 7,85 (d, 1H, J = 9,0 Hz).

### EXEMPLE 29 : 7-[(6-déoxy-3-O-(((1,1-diméthyléthyl) amino) carbonyl)-5-C-méthyl-4-O-méthyl-alpha-L-lyxo-hexopyranosyl) oxy]-4-hydroxy-8-méthyl-2H-1-benzopyran-3-carboxylate d'éthyle

rf = 0;37 CH₂Cl₂-CH₃OH (92-8)
RMN du proton (300 MHz, DMSO-d₆, ppm) δ
1,02 (s, 3H), 1,26 (s, 12H), 1,32 (t, 3H, J = 7,0 Hz), 2,19 (s, 3H), 3,46 (s, 3H), 4,06 (sl, 1H), 4,37 (q, 2H, J = 7,0 Hz), 5,17 (dd, 1H, J = 3,0 et 10,0 Hz), 5,58 (d, 1H, J = 2,5 Hz), 5,62 (sl, 1H), 7,03 (sl, 1H), 7,19 (d, 1H, J = 9,0 Hz), 7,85 (d, 1H, J = 9,0 Hz).

### EXEMPLE 30 : 7-[(3-O-((cyclohexylamino) carbonyl)-6-déoxy-5-C-méthyl-4-O-méthyl-alpha-L-lyxo-hexopyranosyl) oxy]-4-hydroxy-8-méthyl-2H-1-benzopyran-3-carboxylate d'éthyle

rf = 0,33 CH₂Cl₂-CH₃OH (92-8)
RMN du proton (300 MHz, DMSO-d₆, ppm) δ
1,00-1,80 (m, 10H), 1,03 (s, 3H), 1,26 (s, 3H), 1,32 (t, 3H, J = 7,0 Hz), 2,18 (s, 3H), 3,46 (s, 3H), 3,63 (d, 1H, J = 10,0 Hz), 4,08 (sl, 1H), 4,37 (q, 2H, J = 7,0 Hz), 5,20 (dd, 1H, J = 3,0 et 10,0 Hz), 5,61 (d, 1H, J = 2,0 Hz), 5,65 (sl, 1H), 7,19 (d, 1H, J = 9,0 Hz), 7,25 (d, 1H, J = 8,0 Hz), 7,86 (d, 1H, J = 9,0 Hz).

### EXEMPLE 31 : 7-[(6-déoxy-5-C-méthyl-4-O-méthyl-3-O-((4-méthyl-1-pipérazinyl) carbonyl)-alpha-L-lyxo-hexopyranosyl) oxy]-4-hydroxy-8-méthyl-2H-l-benzopyran-3-carboxylate d'éthyle

rf = 0,22 CH₂Cl₂-CH₃OH (85-15)
RMN du proton (300 MHz, DMSO-d₆, ppm) δ
1,02 (s, 3H), 1,20 (t, 3H, J = 7,0 Hz), 1,30 (s, 3H), 2,12 (s, 3H), 2,60 (sl, 3H), 3,04 (1, 8H), 3,44 (s, 3H), 3,54 (d, 1H, J = 10 Hz), 4,05 (m, 3H), 5,4 (dd, 1H, J = 3 et 10 Hz), 5,50 (d, 1H, J = 2,0 Hz), 5,62 (d, 1H, J = 4,5 Hz), 6,93 (d, 1H, J = 9,0 Hz), 7,65 (d, 1H, J = 9,0 Hz).

### EXEMPLE 32 : (Z) 7-[(6-déoxy-5-C-méthyl-4-O-méthyl-2,3-O-(1-méthyléthylidène)-alpha-L-lyxo-hexopyranosyl) oxy]-4-hydroxy-3-(1-hydroxy-2-(2-pyridinyl) éthényl)-8-méthyl-2H-1-benzopyran-2-one

On ajoute 611 mg de chlorhydrate d'acide pyridylacétique et 1,29 mg de 4-diméthylaminopyridine dans une solution renfermant 1,452 g de (Z) 7-[(6-déoxy-5-C-méthyl-4-O-méthyl-2,3-O-(1-méthyléthylidène)-alpha-L-lyxo-hexopyranosyl) oxy]4-hydroxy-8-méthyl-2H-1-benzopyran-2-one et 15 ml de dichlorométhane anhydre. On ajoute ensuite 606 *µ*l de diisopropylcarbodiimide. On maintient sous agitation pendant 20 heures. On ajoute 305,5 mg de chlorhydrate d'acide pyridylacétique et 0,645 mg de 4-diméthylaminopyridine. On agite à nouveau pendant 12 heures. On dilue avec 250 ml de dichlorométhane. On lave avec-70 ml d'une solution aqueuse de dihydrogénophosphate de sodium 1N. On lave à l'eau, à la saumure, sèche, filtre et évapore à sec. On obtient 4,06 g de produit que l'on reprend dans l'éther éthylique à 0°C. On essore et obtient 1,594 g de produit que l'on purifie par chromatographie sur silice en éluant avec le mélange CH₂Cl₂-MeOH (96,5-3,5). On obtient 1,54 g d'un produit que l'on triture dans l'éthanol absolu, refroidit à -10°C. On essore et sèche. On concentre à sec les eaux mères et obtient 0,317 g de produit que l'on triture dans 5 ml d'éthanol auquel on ajoute 4 ml d'éther sulfurique. Après isolation et séchage, on obtient 287 mg de produit recherché.
rf = 0,40 CH₂Cl₂-CH₃OH (95-5)

### EXEMPLE 33 : (Z) 7-[(6-déoxy-5-C-méthyl-4-O-méthyl-alpha-L-lyxo-hexopyranosyl) oxy]-4-hydroxy-3-(1-hydroxy-2-(2-pyridinyl) éthényl)-8-méthyl-2H-1-benzopyran-2-one

On ajoute à 0°C, 1,15 g du produit préparé à l'exemple précédent dans 5 ml d'une solution d'acide trifluoroacétique contenant 10 % d'eau. On agite 4 minutes à 0°C puis ajoute 80 ml de solution tampon constituée par 40 ml de KH₂PO₄ 2M, et 40 ml de K₂HPO₄ 2M. On isole le précipité. On reprend au dichlorométhane et au tétrahydrofuranne. On lave avec une solution de bicarbonate de sodium à 10 % puis à la saumure et on sèche. On évapore les solvants et recueille 1,09 g de produit que l'on disperse dans 4 ml d'éthanol absolu. On isole l'insoluble par filtration. On rince à l'éthanol. On sèche et recueille 291 mg de produit recherché.

### EXEMPLE 34 : (Z) acide carbamique 3'-ester de 7-[(6-déoxy-5-C-méthyl-4-O-méthyl-alpha-L-lyxo-hexopyranosyl) oxy]-4-hydroxy-3-(1-hydroxy-2-(2-pyridinyl) éthényl)-8-méthyl-2H-1-benzopyran-2-one

### STADE A : (Z) 7-[(2,3-O-carbonyl-6-déoxy-5-C-méthyl-4-O-méthyl-alpha-L-lyxo-hexopyranosyl) oxy]-4-hydroxy-3-(1-hydroxy-2-(2-pyridinyl) éthényl)-8-méthyl-2H-1-benzopyran-2-one

On ajoute 84 mg de carbonyldiimidazole dans une solution renfermant 210 mg du produit de l'exemple précédent et 10 ml de tétrahydrofuranne. On porte au reflux pendant 1 heure, ajoute 40 mg-de carbonyidiimidazole. On chauffe encore 1 heure au reflux et dilue à l'acétate d'éthyle. On lave avec une solution de phosphate acide de sodium 1M, puis avec la saumure. On sèche, filtre et concentre à sec. On sèche et obtient 107 mg de produit recherché.

### STADE B : (Z) acide carbamique 3'-ester de 7-[(6-déoxy-5-C-méthyl-4-O-méthyl-alpha-L-lyxo-hexopyranosyl) oxy]-4-hydroxy-3-(1-hydroxy-2-(2-pyridinyl) éthényl)-8-méthyl-2H-1-benzopyran-2-one

On condense environ 15 ml d'ammoniac dans un ballon. On ajoute 107 mg du produit obtenu au stade A. On agite la solution réactionnelle au reflux de l'ammoniac pendant 30 minutes. On laisse l'ammoniac s'échapper. On verse le produit obtenu dans 100 ml de tétrahydrofuranne en présence de 50 ml de solution aqueuse à 10 % d'hydrogénosulfate de sodium. On lave la solution organique à la saumure et sèche. On filtre et évapore à sec. On triture le produit obtenu sous ultrasons avec de l'éther isopropylique. On essore et sèche. On obtient 89 mg de produit recherché.
rf = 0,27 CH₂Cl₂CH₃OH (90-10)
RMN du proton (300 MHz, DMSO-d₆, ppm) δ
1,05 (s, 3H), 1,26 (s, 3H), 2,19 (s, 3H), 3,47 (s, 3H), 3,50 (1, 1H), 4,07 (sl, 1H), 5,15 (dd, 1H, J = 3,0 et 10,0 Hz), 5,52 (d, 1H, J = 2,5 Hz), 5,60 (sl, 1H), 6,50-6,70 (1, 2H), 6,82 (s, 1H), 7,05 (t, 1H, J = 8,0 Hz), 7,09 (d, 1H, J = 9,0 Hz), 7,63 (m, 1H), 7,79 (d, 1H, J = 9,0 Hz), 7,92 (tl, 1H, J = 7,5 Hz), 8,16 (t, 1H, J = 6,0 Hz), 13,81 (sl, 1H), 16,0 (sl, 1H).

### EXEMPLE 35 : acide 5-méthyl-lH-pyrrol-2-carboxylique 3'-ester de 7-((6-déoxy-5-C-méthyl-4-O-méthyl-alpha-L-lyxo-hexopyranosyl) oxy]-4-hydroxy-8-méthyl-2-oxo-2H-1-benzopyran-3-carboxamide

### Stade A : acide 5-méthyl-1H-pyrrole-2-carboxylique 3'-ester de 7-[(6-déoxy-5-C-méthyl-4-O-méthyl-2-O-(tétrahydro-2H-pyran-2-yl)-alpha-L-lyxo-hexopyranosyl)oxy]-8-méthyl-2-oxo-4-(phénylméthoxy)-2H-1-benzopyran-3-carboxylate d'éthyle

On ajoute 1,3 ml de 1,2-dihydropyranne puis 70 mg d'acide paratoluènesulfonique dans une solution renfermant 4,51 g du produit du stade A de l'exemple 4. On agite à la température ambiante pendant 1 h 30 minutes, lave au carbonate de sodium, sèche sur sulfate de magnésium et filtre. On purifie par chromatographie sur silice en éluant avec le mélange hexane-actétate d'éthyle 7-3. On obtient 1,92 g de produit recherché. rf = 0,38 hexane-acétate d'éthyle (1-1).

### Stade B : acide 5-méthyl-1H-pyrrole-2-carboxylique 3'-ester de 7-[(6-déoxy-5-C-méthyl-4-O-méthyl-2-O-(tétrahydro-2H-pyran-2-yl)-alpha-L-lyxo-hexopyranosyl)oxy]-4-hydroxy-8-méthyl-2-oxo-2H-1-benzopyran-3-carboxylate d'éthyle

On ajoute 200 mg de palladium sur charbon dans une solution renfermant 1,92 g du produit du stade A et 25 ml de tétrahydrofuranne. On agite à la température ambiante sous pression d'hydrogène pendant 1 heure. On filtre pour éliminer le catalyseur et évapore le solvant à sec. On dissout dans l'éther éthylique, précipite à l'hexane, évapore à sec le solvant et sèche sous pression réduite. On obtient 1,629 g de produit rf = 0,30 hexane-acétate d'éthyle (1-4).

### Stade C : acide 5-méthyl-1H-pyrrol-2-carboxylique 3'-ester de 7-((6-déoxy-5-C-méthyl-4-O-méthyl-alpha-L-lyxo-hexopyranosyl) oxy]-4-hydroxy-8-méthyl-2-oxo-2H-1-benzopyran-3-carboxamide

Une solution de 350 mg du produit du stade précédent dans 10 ml de tétrahydrofuranne est saturée en ammoniac à 0°C (par barbotage d'ammoniac). On agite la solution réactionnelle à la température ambiante pendant 2 jours. On dilue avec 50 ml d'un mélange hexane-acétate d'éthyle (1-4), lave avec 50 ml d'une solution aqueuse d'hydrogénosulfate de sodium à 10 %. On sèche et concentre à sec. On dissout le produit obtenu (340 mg) dans 10 ml de méthanol et 10 ml de dichlorométhane. On ajoute 50 mg d'acide paratoluènesulfonique monohydrate. On agite 3 heures à la température ambiante. On dilue avec 70 ml de dichlorométhane et lave avec une solution aqueuse saturée de bicarbonate de sodium. On sèche et concentre à sec. On triture en présence d'éther isopropylique. On essore l'insoluble et recueille 170 mg de produit recherché.
RMN (300 MHz, DMSO-d₆, ppm) δ
1,07 (s, 3H), 1,30 (s, 3H), 2,23 (s, 3H), 2,24 (s, 3H), 3,47 (s, 3H), 3,66 (d, 1H, J = 10 Hz), 4,18 (sl, 1H,), 5,48 (dd, 1H, J = 3 et 10 Hz), 5,66 (d, 1H, J = 2,5 Hz), 5,74 (sl, 1H), 5,93 (t, 1H, J = 3 Hz), 6,78 (t, 1H, J = 3 Hz), 7,24 (d, 1H, J = 9 Hz), 7,85 (d, 1H, J = 9 Hz), 8,73 (sl, 1H), 8,98 (sl, 1H), 11,66 (sl, 1H), 13,50 (sl, 1H).

### EXEMPLE 36 : acide 5-méthyl-lH-pyrrol-2-carboxylique 3'-ester de 3-(cyclopropylcarbonyl) 7-((6-déoxy-5-C-méthyl-4-O-méthylalpha-L-lyxo-hexopyranosyl) oxy]-4-hydroxy-8-méthyl-2H-1-benzopyran-2-one

On ajoute 78 *µ*l d'acide cyclopropylcarboxylique dans une solution renfermant 400 mg du produit du stade C de l'exemple 1, 173 mg de chlorhydrate de N-(3-déméthyl-aminopropyl)-N'-éthyl-carbodiimide et 306 mg de 4-diméthylaminopyridine dans du dichlorométhane, on ajoute 78 *µ*l d'acide cyclopropylcarboxylique. On agite 5 heures à la température ambiante. On ajoute à nouveau 86 mg de chlorhydrate de N-(3-diméthylaminopropyl)-N'-éthylcarbodiimide et 159 mg de 4-diméthylaminopyridine. On agite pendant 1 nuit à la température ambiante. On dilue avec 50 ml de dichlorométhane et lave avec 50 ml d'une solution aqueuse d'hydrogénosulfate de sodium à 10 %. On sèche. On évapore à sec les solvants. On obtient 408 mg de produit (rf = 0,78 CH₂Cl₂-MeOH 94-6). On dissout le produit dans un mélange de 15 Ml de méthanol et 10 ml de dichlorométhane. On ajoute 60 mg d'acide paratoluènesulfonique et agite 3 heures à la température ambiante. On dilue au dichlorométhane, lave avec une solution aqueuse de bicarbonate de sodium. On sèche sur sulfate de magnésium et concentre à sec. On purifie par chromatographie sur silice en éluant CH₂Cl₂-MeOH (94-6). On met le produit en solution dans 1 ml d'éther éthylique puis précipite par ajout de 5 ml de n-pentane. On essore et sèche. On obtient 139 mg de produit recherché.
RMN (300 MHz, DMSO, ppm) δ
1,07 (s, 3H), 1,30 (s, 3H), 1,24 (sl, 2H), 1,27 (sl, 2H), 2,24 (s, 3H), 2,25 (sl, 3H), 3,48 (s, 3H), 3,58 (m, 1H), 3,66 (d, 1H, J = 10 Hz), 4,18 (1, 1H), 5,48 (dd, 1H, J = 3 et 10 Hz), 5,68 (d, 1H, J = 2,5 Hz), 5,75 (d, 1H, J = 5 Hz), 5,93 (t, 1H, J = 3 Hz), 6,79 (t, 1H, J = 3 Hz), 7,24 (d, 1H, J = 9 Hz), 7,91 (d, 1H, J = 9 Hz), 11,67 (s, 1H), 13,88 (1, 1H).

### EXEMPLES DE COMPOSITIONS PHARMACEUTIOUES

On a préparé des comprimés renfermant :

| | |
|---|---|
| **Produit de l'exemple 4** | 150 mg |
| Excipient q.s.p. Détail de l'excipient : amidon, talc, stéarate de magnésium | 1 g |
| **Produit de l'exemple 5** | 150 mg |
| Excipient q.s.p. Détail de l'excipient : amidon, talc, stéarate de magnésium | 1 g |
| **Produit de l'exemple 2** | 150 mg |
| Excipient q.s.p. | 1 g |

Détail de l'excipient : amidon, talc, stéarate de magnésium

On a également préparé des solutions injectables à partir des salifiés.

### ETUDE PHARMACOLOGIOUE DES PRODUITS DE L'INVENTION

### A - Méthode des dilutions en milieu liquide

On a préparé une série de tubes dans lesquels on répartit une même quantité de milieu nutritif stérile. On distribue dans chaque tube des quantités croissantes du produit à étudier, puis chaque tube est ensemencé avec une souche bactérienne. Après incubation de vingt-quatre heures à l'étuve à 37°C, l'inhibition de la croissance est appréciée par transillumination de ce qui permet de déterminer les concentrations minimales inhibitrices (C.M.I.) exprimées en microgrammes/cm³.
Sur les souches suivantes :

| | |
|---|---|
| S. aureus | 011HT3 |
| S. aureus | 011UC4 |
| S. aureus | 011HT28 |
| S. epidermidis | 012GO20 |
| S. aureus | 011DU5 |
| S. aureus | 011CB20 |
| S. aureus | 011HT26 |
| S. epidermidts | 012GO39 |
| S. epidermidis | 012HI1 |
| Staph. coag. négative | 012HT5 |
| Staph. coag. négative | 014HI1 |
| S. pyogenes | 02A1UC1 |

Les résultats suivants ont été obtenus :
0,04 < CMI < 20

### B - Inhibition de la gyrase B

Les produits sont des inhibiteurs de gyrase B ; la dose à 50 % du surenroulement de l'ADN est inférieure à 5 *µ*g/ml.

## Revendications

1. Les composés de formule (I) : 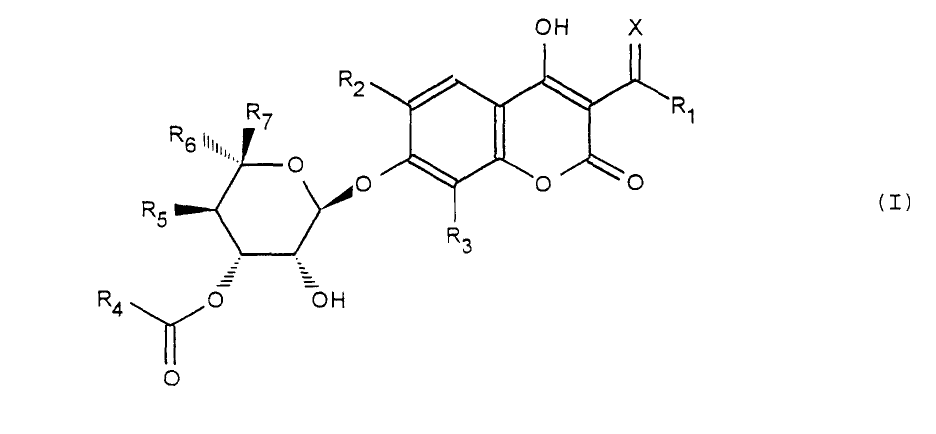 dans laquelle :
R₁ représente un atome d'hydrogène, un radical hydroxyle, un radical alkyle, alkényle ou alkynyle éventuellement interrompu par un atome d'oxygène, de soufre ou d'azote, renfermant jusqu'à 12 atomes de carbone linéaire, ramifié ou cyclique, éventuellement substitué par un ou plusieurs atomes d'halogène, un ou plusieurs radicaux OH, C≡N, NO₂, 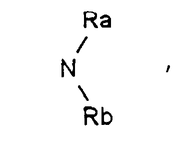 dans lequel Ra et Rb, identiques ou différents, représentent un atome d'hydrogène, un radical alkyle renfermant jusqu'à 8 atomes de carbone, ou Ra et Rb forment avec l'atome d'azote auquel ils sont liés un hétérocycle renfermant éventuellement un autre hétéroatome choisi parmi l'azote, le soufre ou l'oxygène, ou R₁ représente un radical alkoxy renfermant jusqu'à 8 atomes de carbone, éventuellement substitué par un ou plusieurs des substituants indiqués ci-dessus,
ou R₁ représente un radical NRcRd dans lequel Rc et Rd, identiques ou différents représentent un atome d'hydrogène ou un radical alkyle renfermant jusqu'à 12 atomes de carbone, éventuellement substitué par un ou plusieurs des substituants indiqués ci-dessus, ou Rc et Rd forment ensemble avec l'atome d'azote auquel ils sont liés un hétérocycle renfermant éventuellement un autre hétéroatome choisi parmi l'azote, le soufre ou l'oxygène,
X représente un atome d'oxygène, ou un radical N-Nalc₁ ou NOalc₂ dans lequel alc₁ et alc₂ représentent un radical alkyle éventuellement interrompu par un atome d'oxygène, de soufre ou d'azote, renfermant jusqu'à 12 atomes de carbone éventuellement substitué par un ou plusieurs atomes d'halogène, par un ou plusieurs radicaux 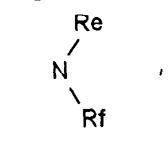 dans lequel Re et Rf, identiques ou différents l'un de l'autre représentent un atome d'hydrogène, un radical alkyle renfermant jusqu'à 8 atomes de carbone, éventuellement substitué, ou Re et Rf pouvant former ensemble avec l'atome d'azote auquel ils sont liés un hétérocycle pouvant renfermer en outre un atome d'oxygène, de soufre et un autre atome d'azote,
R₂ représente un atome d'hydrogène ou un atome d'halogène,
R₃ représente un atome d'hydrogène, un radical alkyle, renfermant jusqu'à 8 atomes de carbone ou un atome d'halogène,
R₄ représente un radical 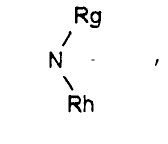 dans lequel Rg et Rh identiques ou différents l'un de l'autre, représentent un atome d'hydrogène, un radical alkyle linéaire, ramifié, cyclique, renfermant jusqu'à 8 atomes de carbone, un radical aryle ou hétéroaryle, éventuellement substitué, ou Rg et Rh forment avec l'atome d'azote auquel ils sont liés un hétérocycle pouvant renfermer en outre un atome d'oxygène, de soufre et un autre atome d'azote, ou R₄ représente un radical aryle ou hétéroaryle éventuellement substitué par un ou plusieurs atomes d'halogène, un ou plusieurs radicaux hydroxyle, un ou plusieurs radicaux alkyle ou alkoxy renfermant jusqu'à 8 atomes de carbone,
R₅ représente un atome d'hydrogène, un radical O-alkyle renfermant jusqu'à 4 atomes de carbone,
R₆ un radical alkyle ou CH₂-O-alkyle, dans lequel alkyle représente un radical alkyle renfermant jusqu'à 8 atomes de carbone,
R₇ représente un atome d'hydrogène ou un radical alkyle renfermant jusqu'à 8 atomes de carbone, ainsi que les sels des composés de formule (I).

2. Les composés de formule (I) tels que définis à la revendication 1 dans lesquels R₂ représente un atome d'hydrogène.

3. Les composés de formule (I) tels que définis à la revendication 1 ou 2, dans lesquels R₃ représente un radical méthyle.

4. Les composés de formule (I) tels que définis à l'une quelconque des revendications 1 à 3, dans lesquels R₆ représente un radical méthyle.

5. Les composés de formule (I) tels que définis à l'une quelconque des revendications 1 à 4, dans lesquels R₇ représente un atome d'hydrogène ou un radical méthyle.

6. Les composés de formule (I) tels que définis à l'une quelconque des revendications 1 à 5, dans lesquels R₅ représente un radical OCH₃.

7. Les composés de formule (I) tels que définis à l'une quelconque des revendications 1 à 6, dans lesquels R₄ représente un radical

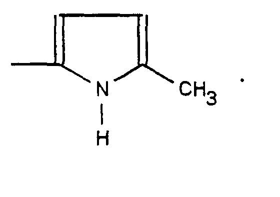

8. Les composés de formule (I) tels que définis à l'une quelconque des revendications 1 à 6, dans lesquels R₄ représente un radical NH-cyclopropyle.

9. Les composés de formule (I) tels que définis à l'une quelconque des revendications 1 à 8, dans lesquels X représente un atome d'oxygène.

10. Les composés de formule (I) tels que définis à l'une quelconque des revendications 1 à 8, dans lesquels X représente un radical NOR, R représentant un radical alkyle, éventuellement substitué par un ou plusieurs atomes d'halogène et éventuellement interrompu par un atome d'oxygène, d'azote, de soufre et portant éventuellement un radical hétérocyclique éventuellement substitué.

11. Les composés de formule (I), tels que définis à la revendication 10, dans lesquels X représente un radical NOCH₃.

12. Les composés de formule (I) tels que définis à l'une quelconque des revendications 1 à 11, dans lesquels R₁ représente un radical alkyle éventuellement interrompu par un atome d'oxygène ou de soufre, un radical O-alkyle, éventuellement interrompu par un atome d'oxygène ou de soufre, un radical NH₂.

13. Les composés de formule (I) tels que définis à la revendication 12, dans lesquels R₁ est un radical CH₃,

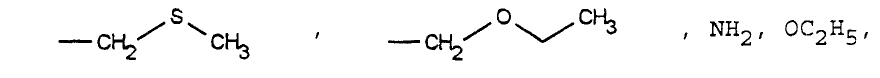

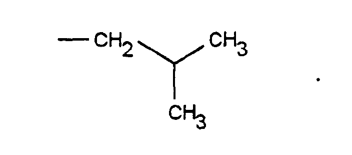

14. Les composés de formule (I) tels que définis à la revendication 13 dans lesquels R₁ représente un radical méthyle ou O-éthyle.

15. Les composés de formule (I) dont les noms suivent :
- l'acide 5-méthyl-1H-pyrrole-2-carboxylique 3'-ester de 3-acétyl-7-((6-déoxy-5-C-méthyl-4-O-méthyl-alpha-L-lyxo-hexopyranosyl) oxy)-4-hydroxy-8-méthyl-2H-1-benzopyran-2-one,
- l'acide 5-méthyl-1H-pyrrole-2-carboxylique 3'-ester de 7-((6-déoxy-5-C-méthyl-4-O-méthyl-alpha-L-lyxo-hexopyranosyl) oxy)-4-hydroxy-8-méthyl-2-oxo-2H-1-benzopyran-3-carboxylate d'éthyle,
- l'acide 5-méthyl-1H-pyrrole-2-carboxylique 3'-ester de 7-((6-déoxy-5-C-méthyl-4-O-méthyl-alpha-L-lyxo-hexopyranosyl) oxy)-4-hydroxy-3-(1-(méthoxyimino) éthyl)-8-méthyl-2H-1-benzopyran-2-one,
- l'acide 5-méthyl-1H-pyrrole-2-carboxylique 3'-ester de 7-((6-déoxy-5-C-méthyl-4-O-méthyl-alpha-L-lyxo-hexopyranosyl) oxy)-3-(éthoxyacétyl)-4-hydroxy-8-méthyl-2H-1-benzopyran-2-one,
- l'acide 5-méthyl-1H-pyrrole-2-carboxylique 3'-ester de 3-(cyclopropylcarbonyl)-7-((6-déoxy-5-C-méthyl-4-O-méthylalpha-L-lyxo-hexopyranosyl) oxy)-4-hydroxy-8-méthyl-2H-1-benzopyran-2-one,
- l'acide 5-méthyl-1H-pyrrole-2-carboxylique 3'-ester de 7-((6-déoxy-5-C-méthyl-4-O-méthyl-alpha-L-lyxo-hexopyranosyl) oxy)-4-hydroxy-8-méthyl-2-oxo-2H-1-benzopyran-3-carboxamide.

16. Procédé de préparation des composés de formule (I), tels que définis à l'une quelconque des revendications 1 à 15 : 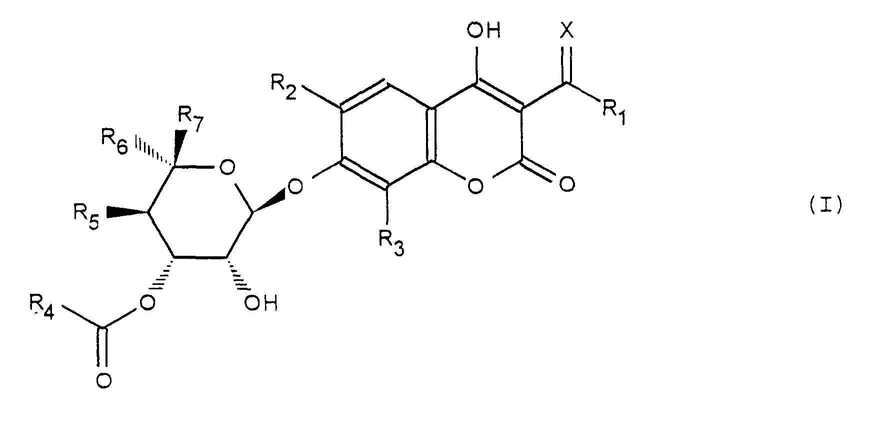 dans lesquels les substituants conservent la même signification que dans la revendication 1, **caractérisé en ce que** l'on soumet un composé de formule (II) : 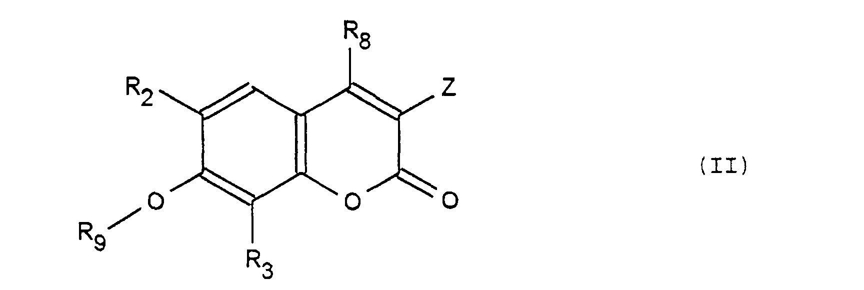 dans laquelle R₈ représente un radical hydroxyle libre ou bloqué, Z représente un atome d'hydrogène ou un radical 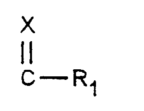 X, R₁, R₂ et R₃ conservent leur signification précédente, OR₉ représente un radical hydroxyle libre ou bloqué,
à l'action d'un composé de formule (III) : 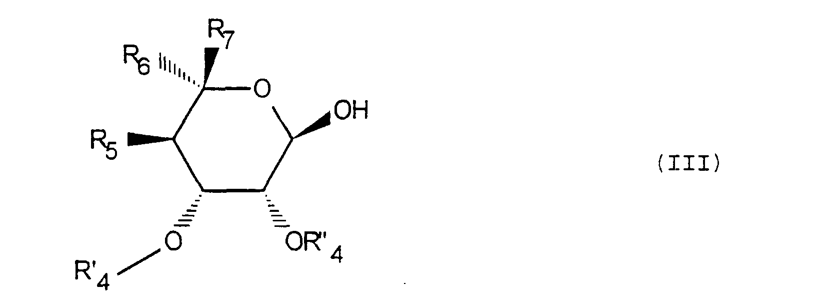 dans lequel R₅, R₆ et R₇ conservent leur signification précédente, OR'₄ représente un radical hydroxyle bloqué, R"₄ représente un atome d'hydrogène ou bien R'₄ et R"₄ forment ensemble avec les atomes de carbone auxquels ils sont joints un cycle 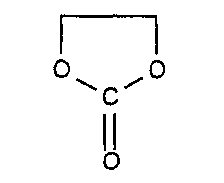 pour obtenir le composé de formule (IV) : 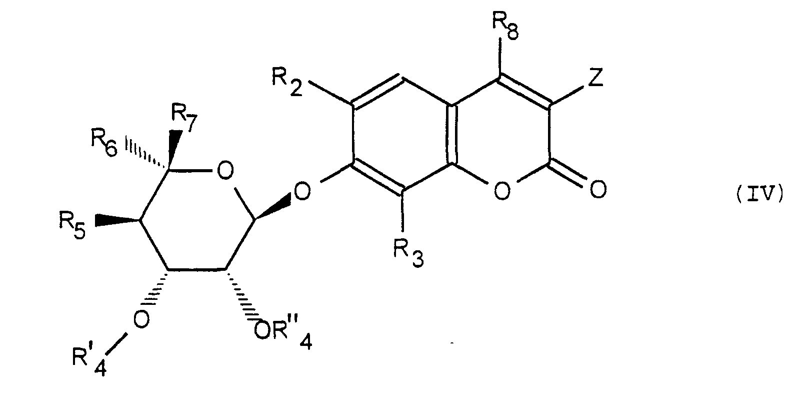 dans lequel les substituants conservent leur signification précédente,
puis soumet le composé de formule (IV) ainsi obtenu aux étapes suivantes, en totalité ou en partie :
- libération de l'hydroxyle en 4 après blocage éventuel de l'hydroxyle du sucre en α de OR'₄,
- si Z représente un atome d'hydrogène introduction du radical 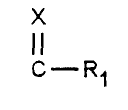 après blocage éventuel des hydroxyles pouvant réagir,
- introduction du radical 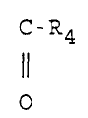 par substitution de ce radical au radical R'₄,
- modification du radical X.

17. A titre de produits chimiques nouveaux les composés de formules (II), (III) et (IV).

18. A titre de médicaments, les composés de formule (I) tels que définis à l'une quelconque des revendications 1 à 15.

19. Les compositions pharmaceutiques renfermant comme principe actif au moins un médicament défini à la revendication 18.

## Patentansprüche

1. Verbindungen der Formel (I): 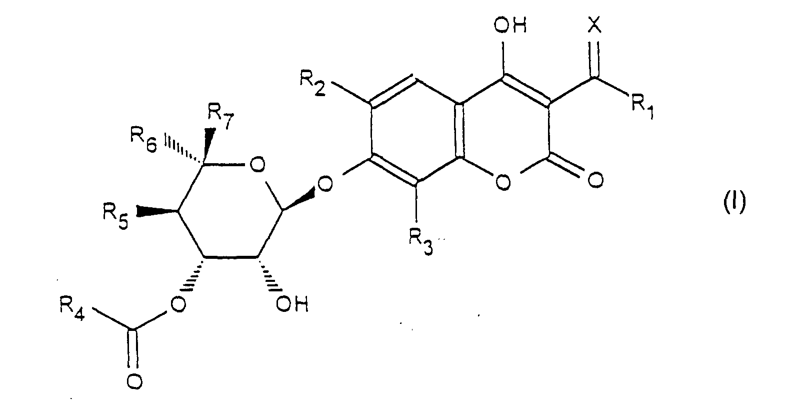 in der:
R₁ ein Wasserstoffatom, einen Hydroxyrest, einen gegebenenfalls durch ein Sauerstoff-, Schwefel- oder Stickstoffatom unterbrochenen linearen, verzweigten oder cyclischen Alkyl-, Alkenyl- oder Alkinylrest mit bis zu 12 Kohlenstoffatomen darstellt, der gegebenenfalls substituiert ist durch ein oder mehrere Halogenatome, einen oder mehrere Reste OH, C≡N, NO₂, 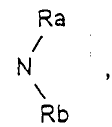 in dem Ra und Rb, gleich oder verschieden, ein Wasserstoffatom, einen Alkylrest mit bis zu 8 Kohlenstoffatomen darstellen oder Ra und Rb mit dem Stickstoffatom, mit dem sie verbunden sind, einen Heterocyclus bilden, der gegebenenfalls ein anderes unter Stickstoff, Schwefel oder Sauerstoff ausgewähltes Heteroatom umfasst, oder R₁ einen Alkoxyrest mit bis zu 8 Kohlenstoffatomen darstellt, der gegebenenfalls durch einen oder mehrere der oben angegebenen Substituenten substituiert ist, oder R₁ einen NRcRd-Rest darstellt, in dem Rc und Rd, gleich oder verschieden, ein Wasserstoffatom oder einen Alkylrest mit bis zu 12 Kohlenstoffatomen darstellen, der gegebenenfalls durch einen oder mehrere der oben angegebenen Substituenten substituiert ist, oder Rc und Rd zusammen mi dem Stickstoffatom, mit dem sie verbunden sind, einen Heterocyclus bilden, der gegebenenfalls ein anderes unter Stickstoff, Schwefel oder Sauerstoff ausgewähltes Heteroatom umfasst,
X ein Sauerstoffatom darstellt oder einen Rest N-Nalc₁ oder NOalc₂, in dem alc₁ und alc₂ einen gegebenenfalls von einem Sauerstoff-, Schwefel- oder Stickstoffatom unterbrochenen Alkylrest mit bis zu 12 Kohlenstoffatomen darstellen, der gegebenenfalls substituiert ist durch ein oder mehrere Halogenatome, durch einen oder mehrere Reste 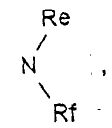 in dem Re und Rf, gleich oder verschieden, jeweils ein Wasserstoffatom, einen gegebenenfalls substituierten Alkylrest mit bis zu 8 Kohlenstoffatomen darstellen, oder Re und Rf zusammen mit dem Stickstoffatom, mit dem sie verbunden sind, einen Heterocyclus bilden können, der außerdem ein Sauerstoff, Schwefel- und ein anderes Stickstoffatom umfassen kann, darstellt,
R₂ ein Wasserstoffatom oder ein Halogenatom darstellt,
R₃ ein Wasserstoffatom, einen Alkylrest mit bis zu 8 Kohlenstoffatomen oder ein Halogenatom darstellt,
R₄ einen Rest 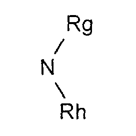 darstellt, in dem Rg und Rh, gleich oder verschieden, jeweils ein Wasserstoffatom, einen linearen, verzweigten, cyclischen Alkylrest mit bis zu 8 Kohlenstoffatomen, einen gegebenenfalls substituierten Aryl- oder Heteroarylrest darstellen, oder Rg und Rh zusammen mit dem Stickstoffatom, mit dem sie verbunden sind, einen Heterocyclus bilden können, der außerdem ein Sauerstoff-, Schwefel- und ein anderes Stickstoffatom umfassen kann, oder R₄ einen Aryl- oder Heteroarylrest darstellt, der gegebenenfalls durch ein oder mehrere Halogenatome, einen oder mehrere Hydroxyreste, einen oder mehrere Alkyl- oder Alkoxyreste mit bis zu 8 Kohlenstoffatomen substituiert ist,
R₅ ein Wasserstoffatom, einen O-Alkyl-Rest mit bis zu 4 Kohlenstoffatomen darstellt,
R₆ einen Alkyl- oder CH₂-O-Alkyl-Rest darstellt, in dem Alkyl einen Alkylrest mit bis zu 8 Kohlenstoffatomen darstellt,
R₇ ein Wasserstoffatom oder einen Alkylrest mit bis zu 8 Kohlenstoffatomen darstellt, sowie die Salze der Verbindungen der Formel (I).

2. Verbindungen der Formel (I) so wie in Anspruch 1 definiert, in denen R₂ ein Wasserstoffatom darstellt.

3. Verbindungen der Formel (I) so wie in Anspruch 1 oder 2 definiert, in denen R₃ einen Methylrest darstellt.

4. Verbindungen der Formel (I) so wie in einem der Ansprüche 1 bis 3 definiert, in denen R₆ einen Methylrest darstellt.

5. Verbindungen der Formel (I) so wie in einem der Ansprüche 1 bis 4 definiert, in denen R₇ ein Wasserstoffatom oder einen Methylrest darstellt.

6. Verbindungen der Formel (I) so wie in einem der Ansprüche 1 bis 5 definiert, in denen R₅ einen OCH₃-Rest darstellt.

7. Verbindungen der Formel (I) so wie in einem der Ansprüche 1 bis 6 definiert, in denen R₄ einen Rest 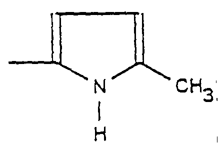 darstellt.

8. Verbindungen der Formel (I) so wie in einem der Ansprüche 1 bis 6 definiert, in denen R₄ einen NH-Cyclopropyl-Rest darstellt.

9. Verbindungen der Formel (I) so wie in einem der Ansprüche 1 bis 8 definiert, in denen X ein Sauerstoffatom darstellt.

10. Verbindungen der Formel (I) so wie in einem der Ansprüche 1 bis 8 definiert, in denen X einen Rest NOR darstellt, wobei R einen Alkylrest darstellt, der gegebenenfalls durch ein oder mehrere Halogenatome substituiert ist und gegebenenfalls durch ein Sauerstoff-, Stickstoff-, Schwefelatom unterbrochen ist und gegebenenfalls einen gegebenenfalls substituierten heterocyclischen Rest trägt.

11. Verbindungen der Formel (I) so wie in Anspruch 10 definiert, in denen X einen NOCH₃-Rest darstellt.

12. Verbindungen der Formel (I) so wie in einem der Ansprüche 1 bis 11 definiert, in denen R₁ einen Alkylrest, der gegebenenfalls durch ein Sauerstoff- oder Schwefelatom unterbrochen ist, einen O-Alkyl-Rest, der gegebenenfalls durch ein Sauerstoff- oder Schwefelatom unterbrochen ist, einen NH₂-Rest darstellt.

13. Verbindungen der Formel (I) so wie in Anspruch 12 definiert, in denen R₁ ein Rest CH₃, 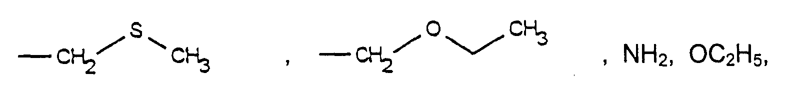 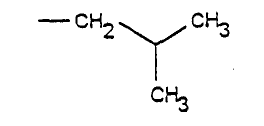 ist.

14. Verbindungen der Formel (I) so wie in Anspruch 13 definiert, in denen R₁ einen Methyl- oder O-Ethyl-Rest darstellt.

15. Verbindungen der Formel (I), deren Namen folgen:
- 5-Methyl-1H-pyrrol-2-carbonsäure-3'-ester von 3-Acetyl-7-((6-deoxy-5-C-methyl-4-O-methyl-alpha-L-lyxo-hexopyranosyl)oxy)-4-hydroxy-8-methyl-2H-1-benzopyran-2-on,
- 5-Methyl-1H-pyrrol-2-carbonsäure-3'-ester von 7-((6-Deoxy-5-C-methyl-4-O-methyl-alpha-L-lyxo-hexopyranosyl)oxy)-4-hydroxy-8-methyl-2-oxo-2H-1-benzopyran-3-ethylcarboxylat,
- 5-Methyl-1H-pyrrol-2-carbonsäure-3'-ester von 7-((6-Deoxy-5-C-methyl-4-O-methyl-alpha-L-lyxo-hexopyranosyl)oxy)-4-hydroxy-3-(1-(methoxyimino)ethyl)-8-methyl-2H-1-benzopyran-2-on,
- 5-Methyl-1H-pyrrol-2-carbonsäure-3'-ester von 7-((6-Deoxy-5-C-methyl-4-O-methyl-alpha-L-lyxo-hexopyranosyl)oxy)-3-(ethoxyacetyl)-4-hydroxy-8-methyl-2H-1-benzopyran-2-on,
- 5-Methyl-1H-pyrrol-2-carbonsäure-3'-ester von 3-(Cyclopropylcarbonyl)-7-((6-deoxy-5-C-methyl-4-O-methyl-alpha-L-lyxo-hexopyranosyl)oxy)-4-hydroxy-8-methyl-2H-1-benzopyran-2-on,
- 5-Methyl-1H-pyrrol-2-carbonsäure-3'-ester von 7-((6-Deoxy-5-C-methyl-4-O-methyl-alpha-L-lyxo-hexopyranosyl)oxy)-4-hydroxy-8-methyl-2-oxo-2H-1-benzopyran-3-carboxamid.

16. Verfahren zur Herstellung von Verbindungen der Formel (I) so wie in einem der Ansprüche 1 bis 15 definiert: 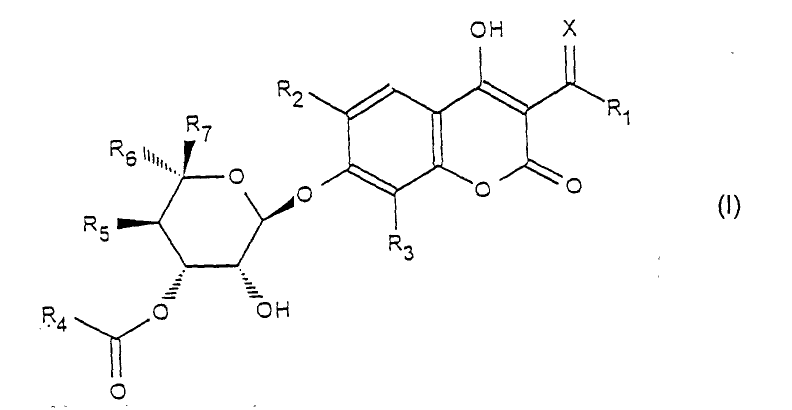 in denen die Substituenten die gleiche Bedeutung wie in Anspruch 1 beibehalten, **dadurch gekennzeichnet, dass** man eine Verbindung der Formel (II): 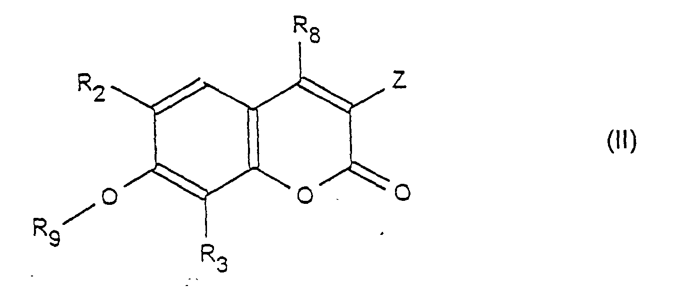 in der R₈ einen freien oder blockierten Hydroxyrest darstellt, Z ein Wasserstoffatom oder einen Rest 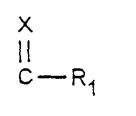 darstellt, X, R₁, R₂ und R₃ ihre vorherige Bedeutung beibehalten, OR₉ einen freien oder blockierten Hydroxyrest darstellt,
der Wirkung einer Verbindung der Formel (III): 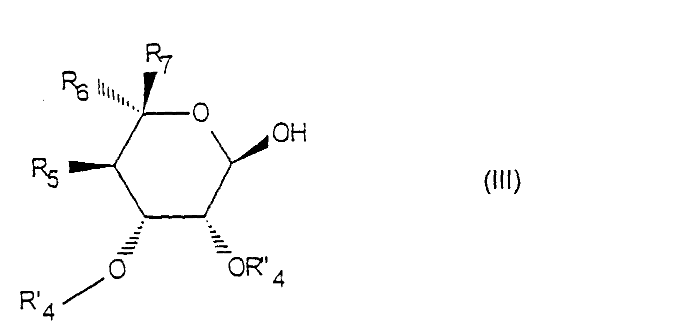 in der R₅, R₆ und R₇ ihre vorherige Bedeutung beibehalten, OR'₄ einen blockierten Hydroxyrest darstellt, R"₄ ein Wasserstoffatom darstellt oder auch R'₄ und R"₄ zusammen mit den Kohlenstoffatomen, mit denen sie verbunden sind, einen Cyclus 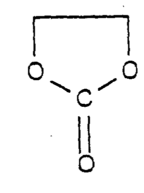 bilden, unterzieht, um die Verbindung der Formel (IV): 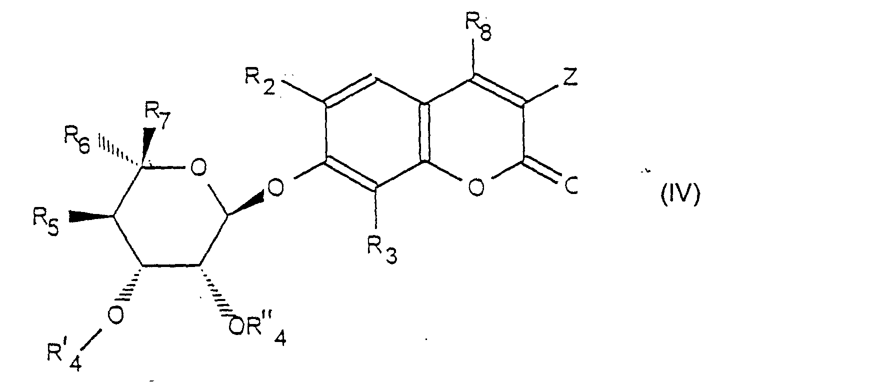 in der die Substituenten ihre vorherige Bedeutung beibehalten, zu erhalten, man dann die so erhaltene Verbindung der Formel (IV) den folgenden Schritten, insgesamt oder teilweise, unterzieht:
- Freisetzung der Hydroxygruppe in 4-Stellung nach eventueller Blockierung der Hydroxygruppe des Zuckers in α-Stellung von OR'₄,
- wenn Z ein Wasserstoffatom darstellt, Einführung des Rests 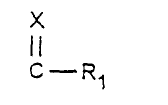 nach eventueller Blockierung von Hydroxygruppen, die reagieren können,
- Einführung des Rests 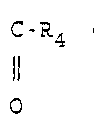 durch Substitution des Rests R'₄ durch diesen Rest
- Modifikation des Rests X.

17. Als neue chemische Produkte die Verbindungen der Formeln (II), (III) und (IV).

18. Als Medikamente die Verbindungen der Formel (I) so wie in einem der Ansprüche 1 bis 15 definiert.

19. Pharmazeutische Zusammensetzungen, die als Wirkstoff wenigstens ein in Anspruch 18 definiertes Medikament umfassen.

## Claims

1. The compounds of formula (I): 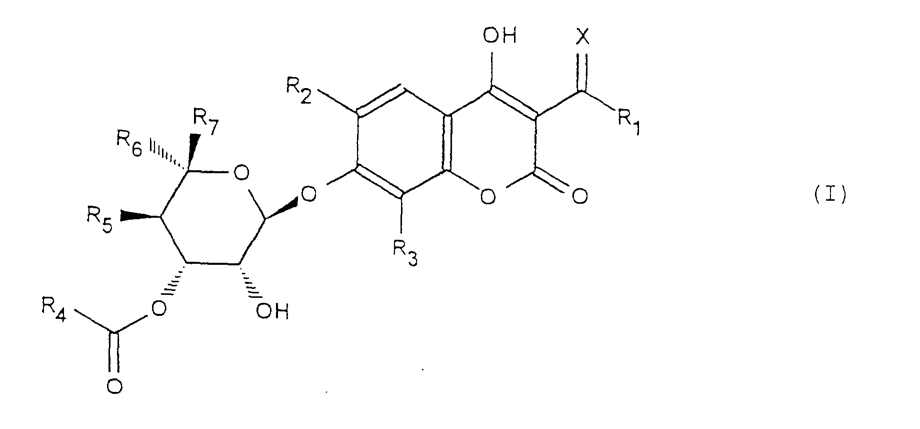 in which:
R₁ represents a hydrogen atom, a hydroxyl radical, an alkyl, alkenyl or alkynyl radical optionally interrupted by an oxygen, sulphur or nitrogen atom, containing up to 12 carbon atoms, linear, branched or cyclic, optionally substituted by one or more halogen atoms, one or more OH, C/N, NO₂, 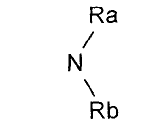 radicals, in which Ra and Rb, identical or different, represent a hydrogen atom, an alkyl radical containing up to 8 carbon atoms, or Ra and Rb form together with the nitrogen atom to which they are linked a heterocycle optionally containing another heteroatom chosen from nitrogen, sulphur or oxygen, or R₁ represents an alkoxy radical containing up to 8 carbon atoms, optionally substituted by one or more of the substituents indicated above,
or R₁ represents an NRcRd radical in which Rc and Rd, identical or different, represent a hydrogen atom or an alkyl radical containing up to 12 carbon atoms, optionally substituted by one or more of the substituents indicated above, or Rc and Rd form together with the nitrogen atom to which they are linked a heterocycle optionally containing another heteroatom chosen from nitrogen, sulphur or oxygen,
X represents an oxygen atom, or an N-Nalk₁ or NOalk₂ radical in which alk₁ and alk₂ represent an alkyl radical optionally interrupted by an oxygen, sulphur or nitrogen atom, containing up to 12 carbon atoms optionally substituted by one or more halogen atoms, by one or more 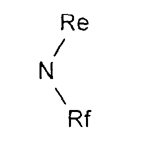 radicals, in which Re and Rf, identical to or different from each other represent a hydrogen atom, an alkyl radical containing up to 8 carbon atoms, optionally substituted, or Re and Rf can form together with the nitrogen atom to which they are linked a heterocycle which can moreover contain an oxygen, sulphur atom and another nitrogen atom,
R₂ represents a hydrogen atom or a halogen atom,
R₃ represents a hydrogen atom, an alkyl radical, containing up to 8 carbon atoms or a halogen atom,
R₄ represents a 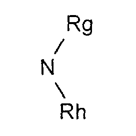 radical, in which Rg and Rh, identical to or different from each other, represent a hydrogen atom, a linear, branched, cyclic alkyl radical, containing up to 8 carbon atoms, an aryl or heteroaryl radical, optionally substituted, or Rg and Rh form together with the nitrogen atom to which they are linked a heterocycle which can moreover contain an oxygen, sulphur atom and another nitrogen atom, or R₄ represents an aryl or heteroaryl radical optionally substituted by one or more halogen atoms, one or more hydroxyl radicals, one or more alkyl or alkoxy radicals containing up to 8 carbon atoms,
R₅ represents a hydrogen atom, an O-alkyl radical containing up to 4 carbon atoms,
R₆ an alkyl or CH₂-O-alkyl radical, in which alkyl represents an alkyl radical containing up to 8 carbon atoms,
R₇ represents a hydrogen atom or an alkyl radical containing up to 8 carbon atoms, as well as the salts of the compounds of formula (I).

2. The compounds of formula (I) as defined in claim 1 in which R₂ represents a hydrogen atom.

3. The compounds of formula (I) as defined in claim 1 or 2, in which R₃ represents a methyl radical.

4. The compounds of formula (I) as defined in any one of claims 1 to 3, in which R₆ represents a methyl radical.

5. The compounds of formula (I) as defined in any one of claims 1 to 4, in which R₇ represents a hydrogen atom or a methyl radical.

6. The compounds of formula (I) as defined in any one of claims 1 to 5, in which R₅ represents an OCH₃ radical.

7. The compounds of formula (I) as defined in any one of claims 1 to 6, in which R₄ represents a 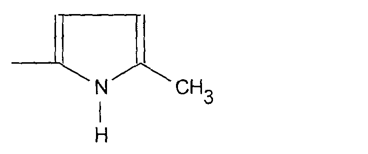 radical.

8. The compounds of formula (I) as defined in any one of claims 1 to 6, in which R₄ represents an NH-cyclopropyl radical.

9. The compounds of formula (I) as defined in any one of claims 1 to 8, in which X represents an oxygen atom.

10. The compounds of formula (I) as defined in any one of claims 1 to 8, in which X represents an NOR radical, R representing an alkyl radical, optionally substituted by one or more halogen atoms and optionally interrupted by an oxygen, nitrogen, sulphur atom and optionally carrying an optionally substituted heterocyclic radical.

11. The compounds of formula (I), as defined in claim 10, in which X represents an NOCH₃ radical.

12. The compounds of formula (I) as defined in any one of claims 1 to 11, in which R₁ represents an alkyl radical optionally interrupted by an oxygen or sulphur atom, an O-alkyl radical, optionally interrupted by an oxygen or sulphur atom, an NH₂ radical.

13. The compounds of formula (I) as defined in claim 12, in which R₁ is a CH₃, 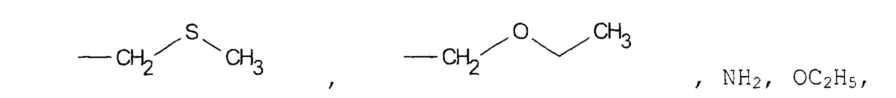 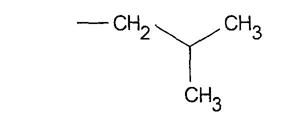 radical.

14. The compounds of formula (I) as defined in claim 13 in which R₁ represents a methyl or O-ethyl radical.

15. The compounds of formula (I) the names of which follow:
- 5-methyl-1H-pyrrole-2-carboxylic acid 3'-ester of 3-acetyl-7-((6-deoxy-5-C-methyl-4-O-methyl-alpha-L-lyxo-hexopyranosyl) oxy)-4-hydroxy-8-methyl-2H-1-benzopyran-2-one,
- 5-methyl-1H-pyrrole-2-carboxylic acid 3'-ester of ethyl 7-((6-deoxy-5-C-methyl-4-O-methyl-alpha-L-lyxo-hexopyranosyl) oxy)-4-hydroxy-8-methyl-2-oxo-2H-1-benzopyran-3-carboxylate,
- 5-methyl-1H-pyrrole-2-carboxylic acid 3'-ester of 7-((6-deoxy-5-C-methyl-4-O-methyl-alpha-L-lyxo-hexopyranosyl) oxy)-4-hydroxy-3-(1-(methoxyimino) ethyl)-8-methyl-2H-1-benzopyran-2-one,
- 5-methyl-1H-pyrrole-2-carboxylic acid 3'-ester of 7-((6-deoxy-5-C-methyl-4-O-methyl-alpha-L-lyxo-hexopyranosyl) oxy)-3-(ethoxyacetyl)-4-hydroxy-8-methyl-2H-1-benzopyran-2-one,
- 5-methyl-1H-pyrrole-2-carboxylic acid 3'-ester 3-(cyclopropylcarbonyl)-7-((6-deoxy-5-C-methyl-4-O-methylalpha-L-lyxo-hexopyranosyl) oxy)-4-hydroxy-8-methyl-2H-1-benzopyran-2-one,
- 5-methyl-1H-pyrrole-2-carboxylic acid 3'-ester of 7-((6-deoxy-5-C-methyl-4-O-methyl-alpha-L-lyxo-hexopyranosyl) oxy)-4-hydroxy-8-methyl-2-oxo-2H-1-benzopyran-3-carboxamide.

16. Process for the preparation of the compounds of formula (I), as defined in any one of claims 1 to 15: 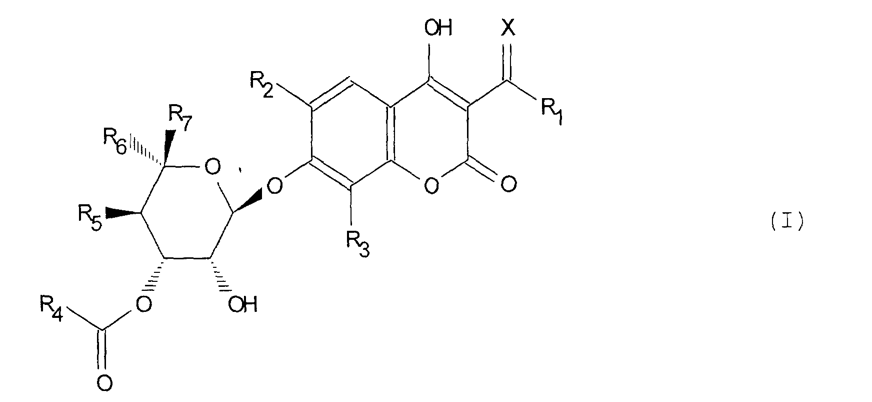 in which the substituents retain the same meaning as in claim 1, **characterized in that** a compound of formula (II): 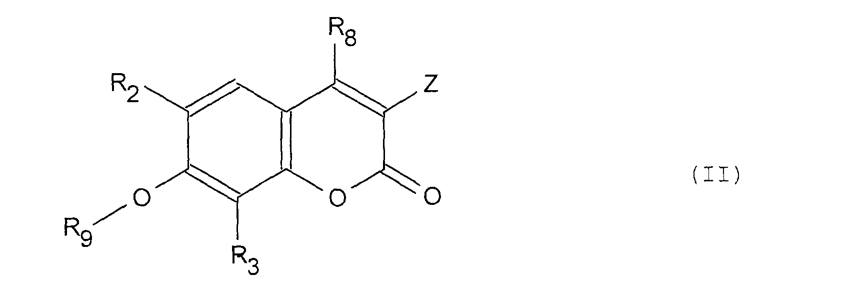 in which R₈ represents a free or blocked hydroxyl radical, Z represents a hydrogen atom or a 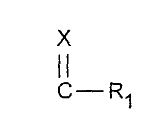 radical
X, R₁, R₂ and R₃ retain their previous meaning, OR₉ represents a free or blocked hydroxyl radical,
is subjected to the action of a compound of formula (III): 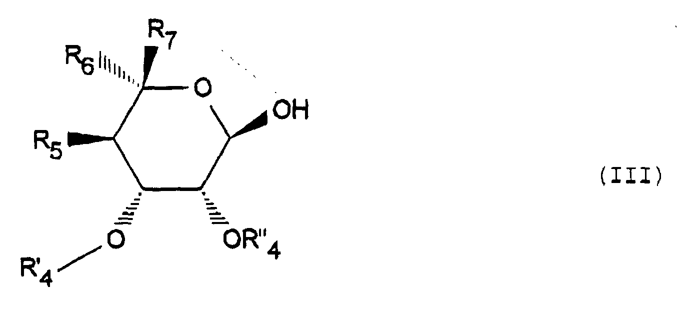 in which R₅, R₆ and R₇ retain their previous meaning, OR'₄ represents a blocked hydroxyl radical, R"₄ represents a hydrogen atom or R'₄ and R"₄ form together with the carbon atoms to which they are joined a 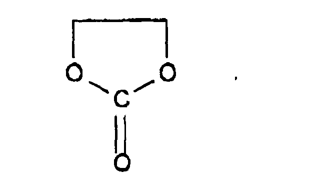 ring, in order to obtain the compound of formula (IV): 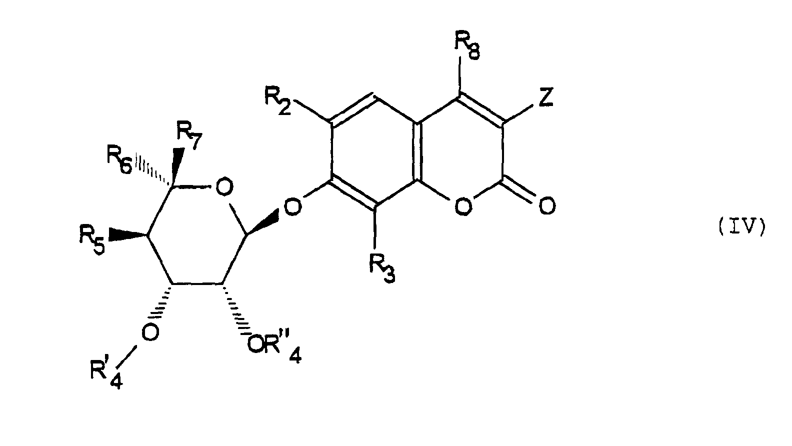 in which the substituents retain their previous meaning, then the compound of formula (IV) thus obtained is subjected to the following stages, totally or partially:
- release of the hydroxyl in position 4 after optional blocking of the hydroxyl of the sugar in α position of OR'₄,
- if Z represents a hydrogen atom, introduction of the 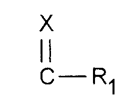 radical after optional blocking of the hydroxyls which can react,
- introduction of the 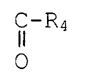 radical by substitution of this radical with the R'₄ radical,
- modification of the X radical.

17. As new chemical products the compounds of formulae (II), (III) and (IV).

18. As medicaments, the compounds of formula (I) as defined in any one of claims 1 to 15.

19. The pharmaceutical compositions containing at least one medicament defined in claim 18 as active ingredient.
